(19) **Europäisches Patentamt / European Patent Office / Office européen des brevets**

(11) **EP 3 916 463 A1**

(12) **EUROPEAN PATENT APPLICATION**
published in accordance with Art. 153(4) EPC

(43) Date of publication:
01.12.2021 Bulletin 2021/48

(51) Int Cl.:
*G02B 23/26* (2006.01)     *G01C 3/06* (2006.01)
*A61B 1/00* (2006.01)     *A61B 1/06* (2006.01)

(21) Application number: 20759296.5

(22) Date of filing: 07.01.2020

(86) International application number:
PCT/JP2020/000149

(87) International publication number:
WO 2020/170621 (27.08.2020 Gazette 2020/35)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 19.02.2019 JP 2019027856

(71) Applicant: Sony Group Corporation
Tokyo 108-0075 (JP)

(72) Inventor: TOMATSU, Kei
Tokyo 108-0075 (JP)

(74) Representative: D Young & Co LLP
120 Holborn
London EC1N 2DY (GB)

(54) **MEDICAL OBSERVATION SYSTEM, MEDICAL SYSTEM AND DISTANCE-MEASURING METHOD**

(57)     A medical observation system includes: an imaging device (2) including an imaging element, and a time-of-flight sensor capable of detecting a distance to a target object; a lens barrel (rigid scope: 3) that includes an illumination optical system and an imaging optical system, and is replaceably mounted to the imaging device (2); and a light source device (4) connected to the illumination optical system of the lens barrel via a light guide. The light source device (4) includes: a first light source (41) configured to emit first light that is guided in the illumination optical system and illuminates a target object; and a second light source (42) configured to emit second light to be detected by the time-of-flight sensor, and the time-of-flight sensor receives the second light that has been reflected by the target object and passed through the imaging optical system of the lens barrel.

*FIG. 1*

# EP 3 916 463 A1

**Description**

TECHNICAL FIELD

The present disclosure relates to a medical observation system, a medical system, and a distance measuring method.

BACKGROUND ART

Conventionally, an endoscope captures an image of a subject by using an imaging device, and observes the subject. Patent Document 1 discloses an imaging device of an endoscope including a time of flight (ToF) measurement light source and a TOF measurement imaging element.

CITATION LIST

PATENT DOCUMENT

Patent Document 1: Japanese Patent Application Laid-Open No. 2017-176811

SUMMARY OF THE INVENTION

PROBLEMS TO BE SOLVED BY THE INVENTION

In the related art described above, when the TOF measurement light source and the TOF measurement imaging element are provided in the imaging device, and an optical system of an endoscope (scope) mounted to the imaging device is reciprocated, a loss occurs in an amount of light received by the TOF measurement imaging element. Furthermore, in order to cope with various subjects, there is a demand for mounting a plurality of types of rigid scopes having different transmittances, lengths, thicknesses, and the like on the imaging device.

Therefore, the present disclosure proposes a medical observation system, a medical system, and a distance measuring method in which a plurality of types of lens barrels can be replaceably mounted to an imaging device including a time-of-flight sensor.

SOLUTIONS TO PROBLEMS

In order to solve the problem described above, a medical observation system according to an aspect of the present disclosure includes: an imaging device including an imaging element, and a time-of-flight sensor capable of detecting a distance to a target object; a lens barrel having an illumination optical system and an imaging optical system and replaceably mounted to the imaging device; and a light source device connected to the illumination optical system of the lens barrel via a light guide. The light source device includes: a first light source configured to emit first light that is guided in the illumination optical system and illuminates the target object; and a second light source configured to emit second light that is guided in the illumination optical system and is to be detected by the time-of-flight sensor, and the time-of-flight sensor receives the second light that has been reflected by the target object and passed through the imaging optical system of the lens barrel.

In order to solve the problem described above, a medical system according to an aspect of the present disclosure includes: a medical observation system including an imaging device including an imaging element, and a time-of-flight sensor capable of detecting a distance to a target object, a lens barrel having an illumination optical system and an imaging optical system and replaceably mounted to the imaging device, and a light source device connected to the illumination optical system of the endoscope via a light guide; and a support arm that has a plurality of links rotatably connected by a joint unit and is configured to be able to hold the imaging device. The light source device includes: a first light source configured to emit first light that is guided in the illumination optical system and illuminates the target object; and a second light source configured to emit second light that is guided in the illumination optical system and is to be detected by the time-of-flight sensor, and the time-of-flight sensor receives the second light that has been reflected by the target object and passed through the imaging optical system of the lens barrel.

Furthermore, a distance measuring method according to an aspect of the present disclosure is a distance calculation method of a medical observation system including: an imaging device including an imaging element, and a time-of-flight sensor capable of detecting a distance to a target object; a lens barrel having an illumination optical system and an imaging optical system and replaceably mounted to the imaging device; and a light source device connected to the illumination optical system of the lens barrel via a light guide. The distance measuring method includes: a step of acquiring an optical distance of each of the illumination optical system and the imaging optical system of the lens barrel, the light guide, the light source device, and the imaging device; a step of causing the light source device to emit light; and a step of calculating a distance from a tip end of the lens barrel to the target object, on the basis of the acquired optical distance and a detection result of the time-of-flight sensor.

BRIEF DESCRIPTION OF DRAWINGS

Fig. 1 is a view showing a schematic configuration of an endoscope system and a medical system according to a first embodiment.
Fig. 2 is a view showing a configuration example of an imaging device according to the first embodiment.
Fig. 3 is a view showing a configuration example of a rigid scope according to the first embodiment.
Fig. 4 is a view showing a configuration example of a light source device according to the first embodiment.
Fig. 5 is a view for explaining an optical path and a distance in the endoscope system according to the first embodiment.
Fig. 6 is a diagram showing an example of a functional configuration of the endoscope system according to the first embodiment.
Fig. 7 is a flowchart showing an example of a processing procedure executed by a control device according to the first embodiment.
Fig. 8 is a view showing an example of optical distance information of a control device according to a modification of the first embodiment.
Fig. 9 is a view showing an example of a configuration of an endoscope system according to a second embodiment.
Fig. 10 is a diagram showing a configuration of a control device according to the second embodiment.
Fig. 11 is a flowchart showing an example of a setting process executed by the control device according to the second embodiment.
Fig. 12 is a flowchart showing an example of a processing procedure executed by the control device according to the second embodiment.
Fig. 13 is a diagram showing a configuration of a control device according to a modification of the second embodiment.
Fig. 14 is a flowchart showing an example of a setting process executed by the control device according to a modification of the second embodiment.
Fig. 15 is a flowchart showing an example of a processing procedure executed by the control device according to a modification of the second embodiment.

MODE FOR CARRYING OUT THE INVENTION

Hereinafter, embodiments of the present disclosure will be described in detail with reference to the drawings. Note that, in each of the following embodiments, the same parts are denoted by the same reference numerals, and redundant description will be omitted.

(First embodiment)

[Outline of endoscope system and medical system according to first embodiment]

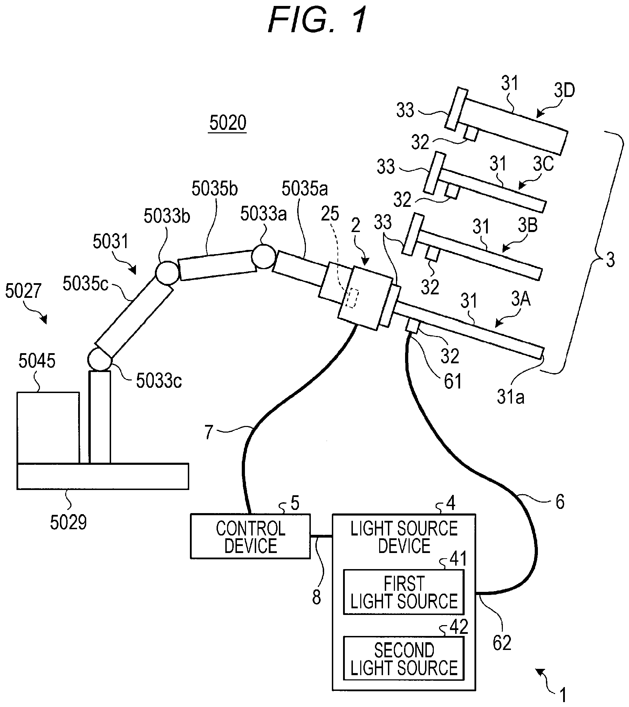

Fig. 1 is a view showing a schematic configuration of an endoscope system and a medical system according to a first embodiment. An endoscope system 1 shown in Fig. 1 is a system that is used in a medical field and observes a subject inside a target object such as a human body. The endoscope system 1 is an example of a medical observation system. For example, the endoscope system 1 has a configuration in which an imaging device 2 is supported by a medical support arm 5027 such that a rigid scope 3 mounted to the imaging device 2 can move with respect to a target object such as a subject. In the present embodiment, the endoscope system 1 is included in a medical system 5020. That is, the medical system 5020 includes the support arm 5027 and the endoscope system 1. The medical system 5020 has a configuration to movably hold, with the support arm 5027, the imaging device 2 of the endoscope system 1 that observes an inside of a body.

[Support arm]

The support arm 5027 has a plurality of links rotatably connected by a joint unit, and is configured to be able to hold the imaging device 2 that observes an inside of the body. For example, the support arm 5027 includes a base unit 5029 that is a base, and an arm unit 5031 extending from the base unit 5029. In the illustrated example, the arm unit 5031 is an articulated arm including a plurality of joint units 5033a, 5033b, and 5033c and a plurality of links 5035a and 5035b connected by the joint unit 5033b. In the example shown in Fig. 1, the configuration of the arm unit 5031 is illustrated in a simplified manner for the sake of simplicity. In practice, a shape, the number, and an arrangement of the joint units 5033a to 5033c and the links 5035a and 5035b, a direction of a rotation axis of the joint units 5033a to 5033c, and the like may be set as appropriate such that the arm unit 5031 has a desired degree of freedom. For example, the arm unit 5031 may be preferably configured to have a degree of freedom of six or more degrees of freedom. With this configuration, since the endoscope system 1 can be freely moved within a movable range of the arm unit 5031, it is possible to insert the rigid scope 3 of the endoscope system 1 into a body cavity of a patient from a desired direction.

For example, the joint units 5033a to 5033c are provided with an actuator, and the joint units 5033a to 5033c are configured to be rotatable around a predetermined rotation axis by driving of the actuator. By controlling the driving of the actuator with an arm control device 5045, rotation angles of the individual joint units 5033a to 5033c are controlled, and driving of the arm unit 5031 is controlled. With this configuration, the support arm 5027 can control a location and a position of the rigid scope 3 of the endoscope system 1. At this time, the arm control device 5045 can control the driving of the arm unit 5031 by various known control methods such as force control or location control. In the present embodiment, the medical system 5020 further includes the arm control device 5045 configured to control the support arm 5027 (articulated arm). The arm control device 5045 includes, for example, a processor such as a central processing unit (CPU), and controls driving of the support arm 5027 in accordance with a predetermined control method, by operating in accordance with a predetermined program. The arm control device 5045 provides a function of controlling the joint units 5033a to 5033c in accordance with a distance from a tip end of the rigid scope 3 to the target object. Furthermore, for example, the support arm 5027 can change the held endoscope to a desired location and position, by having a configuration of appropriately fixing the individual joint units without providing an actuator to the individual joint units 5033a to 5033c.

[Endoscope system]

For example, the endoscope system 1 has a configuration in which a plurality of types of rigid scopes 3 having different light transmittances, lengths, thicknesses, and the like, that is, having different functional and mechanical specifications can be exchanged. In other words, the endoscope system 1 has a configuration in which one rigid scope 3 selected from the plurality of types of rigid scopes 3 is detachably mounted to the imaging device 2.

In the example shown in Fig. 1, the endoscope system 1 includes the imaging device 2, the rigid scope 3, a light source device 4, a control device 5, and a light guide 6.

The imaging device 2 is, for example, a camera head. The imaging device 2 is detachably connected to a mounting unit 33 of the rigid scope 3. The imaging device 2 captures a subject image from the rigid scope 3, and outputs the imaging result. The imaging device 2 outputs the imaging result to the control device 5 via a transmission cable 7. Note that the imaging device 2 may have a configuration of being capable of wirelessly communicating with the control device 5 without using the transmission cable 7.

The imaging device 2 further includes a time-of-flight (TOF) sensor 25 capable of detecting a distance to the target object. The TOF sensor 25 detects a time from when the light source device 4 emits infrared light to when the infrared light is reflected by the target object to be received. In other words, the TOF sensor 25 detects a time-of-flight of the light. The TOF sensor 25 outputs a detection result to the control device 5 via the transmission cable 7.

The rigid scope 3 is a rigid endoscope (scope) to be inserted into a living body. In the example shown in Fig. 1, the rigid scope 3 includes, for example, four types of rigid scopes 3 having different transmittances, lengths, shapes, materials, and the like. Note that the number of types of the rigid scope 3 is not limited to this. Furthermore, in the following description, in a description of distinguishing each of the four types of rigid scopes 3, the four types of rigid scope 3 are appropriately referred to as a rigid scope 3A, a rigid scope 3B, a rigid scope 3C, and a rigid scope 3D.

The rigid scope 3 includes an insertion unit 31, a connection unit 32, and the mounting unit 33. The insertion unit 31 has, for example, an elongated shape, and is inserted into a living body through a natural hole or an artificial hole. The inside of the insertion unit 31 is configured using one or a plurality of lenses, and is provided with an imaging optical system that collects a subject image. The connection unit 32 is detachably connected with one end 61 of the light guide 6, and transmits light supplied from the light source device 4 into the insertion unit 31. The insertion unit 31 irradiates the living body with the light supplied via the connection unit 32, from a tip end 31a of the insertion unit 31. Then, the light (subject image) emitted into the living body is guided to the imaging device 2 by the imaging optical system in the rigid scope 3. The mounting unit 33 is configured to be detachably mounted to the imaging device 2. In the mounting unit 33, light guided through the insertion unit 31 from the tip end 31a of the insertion unit 31 passes toward the imaging device 2.

In the example shown in Fig. 1, the rigid scope 3A has a configuration in which the insertion unit 31 is longer than that of the rigid scope 3B. The rigid scope 3B has a configuration in which the insertion unit 31 is longer than that of the rigid scope 3C. The rigid scope 3C has a configuration having the same length of the insertion unit 31 as that of the rigid scope 3D. The rigid scope 3D has a configuration in which a diameter size of the insertion unit 31 is larger than that of other rigid scopes 3. Then, among the rigid scope 3A, the rigid scope 3B, the rigid scope 3C, and the rigid scope 3D, suitable one for the target object is selected and mounted to the imaging device 2.

The light source device 4 is detachably connected with another end 62 of the light guide 6, and supplies light to be guided by the light guide 6. The light source device 4 is electrically connected to the control device 5 via a transmission cable 8, and light emission (driving) is controlled by control of the control device 5. The light source device 4 is provided outside the imaging device 2 and the rigid scope 3. The light source device 4 includes a first light source 41 and a second light source 42. The first light source 41 emits visible light for illuminating a target object. The light emitted from the first light source 41 is guided inside the light guide 6 and the rigid scope 3, and is emitted from the tip end 31a of the rigid scope 3 toward the target object. The second light source 42 emits infrared light to be detected by the TOF sensor 25. The infrared light emitted from the second light source 42 is guided inside the light guide 6 and the rigid scope 3, and is emitted from the tip end 31a of the rigid scope 3 toward the target object. Then, reflected light of the infrared light is guided in the imaging optical system of the insertion unit 31 of the rigid scope 3 and guided to the TOF sensor 25 in the imaging device 2.

The control device 5 processes various types of information inputted via the transmission cable 7. The control device 5 executes, for example, processing of a causing display device or the like to display an imaging result captured by the imaging device 2. The control device 5 can use, for example, a camera control unit (CCU). The control device 5 executes processing of calculating a distance from a tip end 21a of the rigid scope 3 to the target object, for example, on the basis of a time from when the second light source 42 is caused to emit light to when the TOF sensor 25 detects infrared light. Note that a method of calculating the distance to the target object will be described later. Then, the control device 5 outputs the calculated distance to the arm control device 5045 or the like of the support arm 5027. With this configuration, on the basis of the distance, the arm control device 5045 and the like control a location of the rigid scope 3 of the endoscope system 1 such that the rigid scope 3 does not come into contact with the target object.

One end of the light guide 6 is detachably connected to the light source device 4, and another end is detachably connected to the connection unit 32 of the rigid scope 3. As the light guide 6, for example, an optical fiber or the like can be used. The light guide 6 guides light supplied from the light source device 4 from one end to another end, and supplies the light to the rigid scope 3. The light guide 6 may include, for example, a storage means that stores identification information for identifying the light guide 6, optical distance information indicating an optical distance of the light guide 6, and the like. The storage means may be, for example, a semiconductor memory element such as a RAM or a flash memory. The storage means may be, for example, a pattern of contact pins or the like.

[Configuration of imaging device]

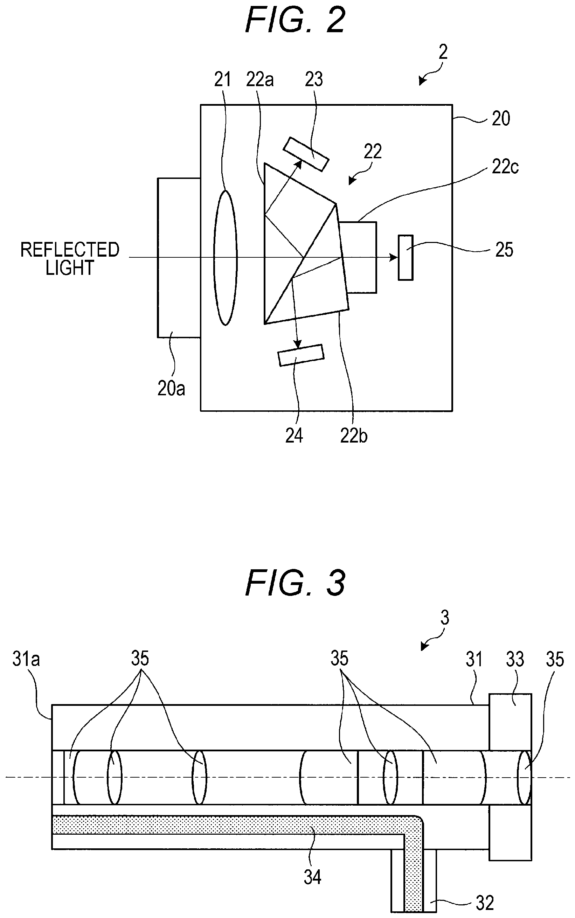

Next, an example of a configuration of the imaging device 2 according to the first embodiment will be described. Fig. 2 is a view showing a configuration example of the imaging device 2 according to the first embodiment. As shown in Fig. 2, the imaging device 2 includes a lens 21, a prism 22, a first imaging element 23, a second imaging element 24, and the TOF sensor 25. The lens 21, the prism 22, the first imaging element 23, the second imaging element 24, and the TOF sensor 25 are housed in a housing 20. The housing 20 has an attachment unit 20a to which the rigid scope 3 is attached. To the housing 20, reflected light of a target object guided in the observation optical system of the rigid scope 3 is made incident.

The lens 21 is a lens that is a part of an observation light source system. The prism 22 includes a first prism 22a, a second prism 22b, and a third prism 22c. The first prism 22a, the second prism 22b, and the third prism 22c are three types of optical prisms, and are joined to each other. In the prism 22, the first prism 22a, the second prism 22b, and the third prism 22c are arranged in this order from a side closer to the lens 21.

In the example shown in Fig. 2, the prism 22 causes a joint surface between the first prism 22a and the second prism 22b and a joint surface between the second prism 22b and the third prism 22c to function as at least one of a beam splitter (BS), a polarizing beam splitter (PBS), or a wavelength selection filter (for example, a dichroic mirror). With this configuration, the prism 22 can distinguish infrared light having passed through the same optical system of the rigid scope 3 and other light from each other.

The first imaging element 23 is an imaging element for visible light observation. The second imaging element 24 is an imaging element for special light observation. The first imaging element 23 and the second imaging element 24 receive reflected light emitted from the first light source of the light source device 4 and reflected by a subject or the like. The first imaging element 23 and the second imaging element 24 have a configuration to receive reflected light having passed through the lens 21, and convert into an electric signal. For the first imaging element 23 and the second imaging element 24, for example, a complementary metal oxide semiconductor (CMOS) image sensor, a charge coupled device (CCD) image sensor, or the like may be used. The first imaging element 23 is arranged in the housing 20 so as to receive reflected light reflected by the joint surface between the first prism 22a and the second prism 22b. The second imaging element 24 is arranged in the housing 20 so as to receive reflected light reflected by the joint surface between the second prism 22b and the third prism 22c. The TOF sensor 25 is arranged in the housing 20 so as to receive reflected light having passed through the first prism 22a, the second prism 22b, and the third prism 22c in this order.

[Configuration of rigid scope]

Next, an example of a configuration of the rigid scope 3 according to the first embodiment will be described. Fig. 3 is a view showing a configuration example of the rigid scope 3 according to the first embodiment. As shown in Fig. 3, the rigid scope 3 includes an insertion unit 31, a connection unit 32, the mounting unit 33, an illumination optical system 34, and an imaging optical system 35. The illumination optical system 34 includes, for example, an optical fiber provided from the tip end 31a of the insertion unit 31 to the connection unit 32. By using the optical fiber, the illumination optical system 34 can guide light from the light guide 6 to the tip end 31a of the insertion unit 31 without loss at other than a connection part with the light guide 6. The illumination optical system 34 can illuminate a subject by emitting light from the tip end 31a of the insertion unit 31.

The imaging optical system 35 is an optical path of the rigid scope 3 through which light reflected by a target object passes. The imaging optical system 35 includes a plurality of lenses. The plurality of lenses includes, for example, lenses of a target objective, a relay system, an eyepiece, and the like. In the example shown in Fig. 3, a description is given to a case where the imaging optical system 35 is provided with seven lenses, but the number of lenses is not limited to this. For example, every time light is transmitted through one lens, the loss becomes 10%, and the transmittance becomes 0.9 lenses $\times$ 100%. Therefore, in the imaging optical system 35 shown in Fig. 3, the transmittance becomes 48% as the light passes through the seven lenses. For example, in a case where the imaging optical system 35 includes fifteen lenses, the transmittance becomes 21%. In the imaging optical system 35, reflected light of the subject having entered from the tip end 31a of the rigid scope 3 is transmitted toward the imaging device 2.

[Configuration of light source device]

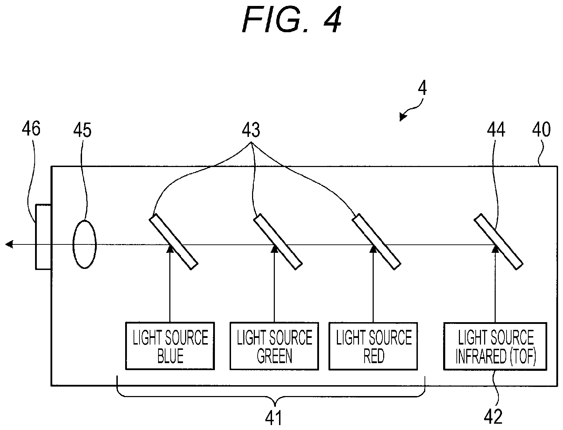

Next, an example of a configuration of the light source device 4 according to the first embodiment will be described. Fig. 4 is a view showing a configuration example of the light source device 4 according to the first embodiment. As shown in Fig. 4, the light source device 4 includes a plurality of first light sources 41, the second light source 42, a plurality of dichroic mirrors 43, a mirror 44, a condenser lens 45, and an emission port 46, which are housed in a housing 40. The plurality of first light sources 41 includes, for example, a light source configured to emit light of red, green, blue, or the like. The second light source 42 includes, for example, a light source configured to emit infrared light. The plurality of dichroic mirrors 43 is arranged so as to individually face the plurality of first light sources 41, and reflects only visible light having a wavelength emitted from the first light source 41, toward the condenser lens 45. The plurality of dichroic mirrors 43 allows light having a wavelength other than a specific wavelength to pass through. The mirror 44 reflects infrared light emitted from the second light source 42, toward the condenser lens 45. The condenser lens 45 condenses light from the plurality of dichroic mirrors 43 and the mirror 44. The emission port 46 emits the light condensed by the condenser lens 45, to the light guide 6. Note that the first light source 41 of the light source device 4 may include, for example, a light source configured to emit light of a color other than red, green, and blue.

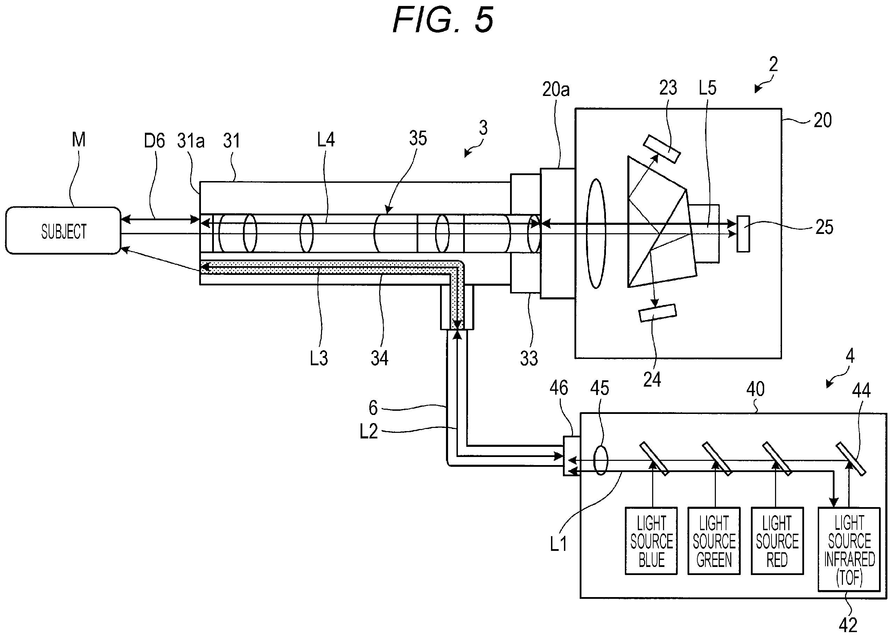

Next, a relationship between an optical path and a distance in a configuration example of the endoscope system 1 according to the first embodiment will be described. Fig. 5 is a view for explaining an optical path and a distance in the endoscope system 1 according to the first embodiment. As shown in Fig. 5, an optical path of the TOF sensor 25 of the endoscope system 1 includes an optical distance L1, an optical distance L2, an optical distance L3, an optical distance L4, an optical distance L5, and a distance D6. The optical distance L1, the optical distance L2, the optical distance L3, the optical distance L4, and the optical distance L5 show individual optical distances of the light source device 4, the light guide 6, the illumination optical system 34 of the rigid scope 3, the imaging optical system 35 of the rigid scope 3, and the imaging device 2. For example, when light passes through a light guide member or the like in a catalyst, an optical distance can be expressed by a product of a refractive index of the catalyst and a passing distance. In other words, the optical distance is a distance that cannot be measured by a caliper or the like.

The optical distance L1 indicates an optical distance from the second light source 42 for the TOF sensor 25 to the emission port 46 in the light source device 4. The optical distance L2 indicates an optical distance in the light guide 6. The optical distance L3 indicates an optical distance of the illumination optical system 34 in the rigid scope 3. The optical distance L4 indicates an optical distance of the imaging optical system 35 in the rigid scope 3. In other words, the optical distance L4 indicates an optical distance from the tip end 31a of the rigid scope 3 to the attachment unit 20a of the imaging device 2. The optical distance L5 indicates an optical distance from the attachment unit 20a to the TOF sensor 25 in the imaging device 2. The distance D6 indicates a distance from the tip end 31a of the rigid scope 3 to the subject (target object).

In the endoscope system 1, light emitted by the light source device 4 is guided in the light guide 6 and the illumination optical system 34 of the rigid scope 3, and is emitted from the tip end 31a of the rigid scope 3 toward a subject M. The subject M is an example of the target object. The light reflected by the subject M enters inside from the tip end 31a of the rigid scope 3, passes through the imaging optical system 35, and is received by the TOF sensor 25 of the imaging device 2. In the endoscope system 1 according to the first embodiment, optical distances on an irradiation side and a reflection side are different. Therefore, there are required the optical distance on the irradiation side over the light source device 4, the light guide 6, and the tip end 31a of the rigid scope 3 and the optical distance on the reflection side.

For example, it is assumed that a speed of light is V, and a time from when the light source device 4 emits light to when the light is received by the TOF sensor 25 is T. In this case, the distance D6 from the tip end 31a of the rigid scope 3 to the subject M can be calculated from the time T from when the light source device 4 emits light to when the light is received by the TOF sensor 25 as a measurement value, and the following Formula (1).

$$D6 = [(V \times T) - (L1 + L2 + L3 + L4 + L5)]/2 \cdots \text{Formula (1)}$$

The control device 5 calculates the distance D6 from the tip end 31a of the rigid scope 3 to the subject M, by using the optical distance L1, the optical distance L2, the optical distance L3, the optical distance L4, the optical distance L5, and Formula (1).

[Operation of endoscope system according to first embodiment]

Next, an example of an operation of the endoscope system 1 according to the first embodiment will be described with reference to Fig. 5. For example, in the endoscope system 1, when the rigid scope 3 is mounted to the imaging device 2, the rigid scope 3 and the light source device 4 are connected by the light guide 6. In the endoscope system 1, the control device 5 causes the first light source 41 and the second light source 42 of the light source device 4 to emit light. The light emitted from the light source device 4 passes through the light guide 6 and enters the rigid scope 3. The light having entered the rigid scope 3 is guided in the illumination optical system 34 of the insertion unit 31 and emitted from the tip end 31a toward the subject M. Since the emitted light includes visible light of the first light source 41, the subject M can be illuminated. Furthermore, reflected light reflected by the subject M enters the imaging optical system 35 from the tip end 31a of the rigid scope 3. The light having entered the imaging optical system 35 passes through the imaging optical system 35 and is made enter the imaging device 2. In the light having entered the imaging device 2, infrared light is received by the TOF sensor 25, and other light is received by the first imaging element 23 and the second imaging element 24. As a result, the TOF sensor 25 can detect a time of the infrared light having passed through the optical distance L1, the optical distance L2, the optical distance L3, the optical distance L4, the optical distance L5, and the distance D6 × 2. Furthermore, the imaging device 2 can obtain an image of the subject M by the light passing through the imaging optical system 35.

As described above, in the endoscope system 1 according to the first embodiment, when a replaced rigid scope 3 is mounted to the imaging device 2, light for illuminating the subject and infrared light to be detected by the TOF sensor 25 are guided in the illumination optical system 34 of the rigid scope 3 via the light guide 6. Then, the endoscope system 1 detects, by the TOF sensor 25, infrared light having been reflected by the subject M and passed through the imaging optical system 35 of the rigid scope 3. With this configuration, in the endoscope system 1, by providing the second light source 42 that emits infrared light for the TOF sensor 25 in the light source device 4 including the first light source 41 for illumination, it is not necessary to provide a light source for the TOF sensor 25 in the imaging device 2, and infrared light for the TOF sensor 25 can be emitted from the illumination optical system 34. As a result, in the endoscope system 1, when the subject M is irradiated with the infrared light for the TOF sensor 25, it is not necessary to pass through the imaging optical system 35 of the rigid scope 3. Therefore, the loss of the infrared light due to the imaging optical system 35 can be suppressed. Furthermore, in the endoscope system 1, even when a plurality of types of the rigid scopes 3 is selectively mounted, a decrease in an amount of infrared light received by the TOF sensor 25 can be suppressed. Therefore, stability of the detection accuracy of the TOF sensor 25 can be improved.

Furthermore, in the endoscope system 1, the control device 5 calculates the distance D6 from the tip end 31a of the rigid scope 3 to the subject M, on the basis of a detection result of the TOF sensor 25. With this configuration, in the endoscope system 1, for example, even when the rigid scope 3 having a different size such as a length and a thickness is mounted to the imaging device 2, the distance D6 from the tip end 31a of the mounted rigid scope 3 to the subject M can be calculated. As a result, in the endoscope system 1, a plurality of different types of the rigid scopes 3 can be mounted to the imaging device 2, which can improve versatility.

Furthermore, in the endoscope system 1, infrared light from the second light source 42 is guided by the rigid scope 3 with the illumination optical system 34, and emitted toward the subject M. With this configuration, in the endoscope system 1, even if the length or the like of the rigid scope 3 is different, the illumination optical system 34 can be made common, or a length of the illumination optical system 34 can be made different. As a result, in the endoscope system 1, since a wide variety of the rigid scopes 3 can be replaced, convenience can be improved.

Furthermore, since the support arm 5027 includes the endoscope system 1, the distance D6 to the subject M can be accurately recognized. With this configuration, even when a plurality of types of the rigid scope 3 is used in the endoscope system 1, the support arm 5027 can control a location of the rigid scope 3 with high accuracy. As a result, since the rigid scope 3 can automatically avoid collision in a body cavity, safety can be improved in the support arm 5027.

[Functional configuration of endoscope system according to first embodiment]

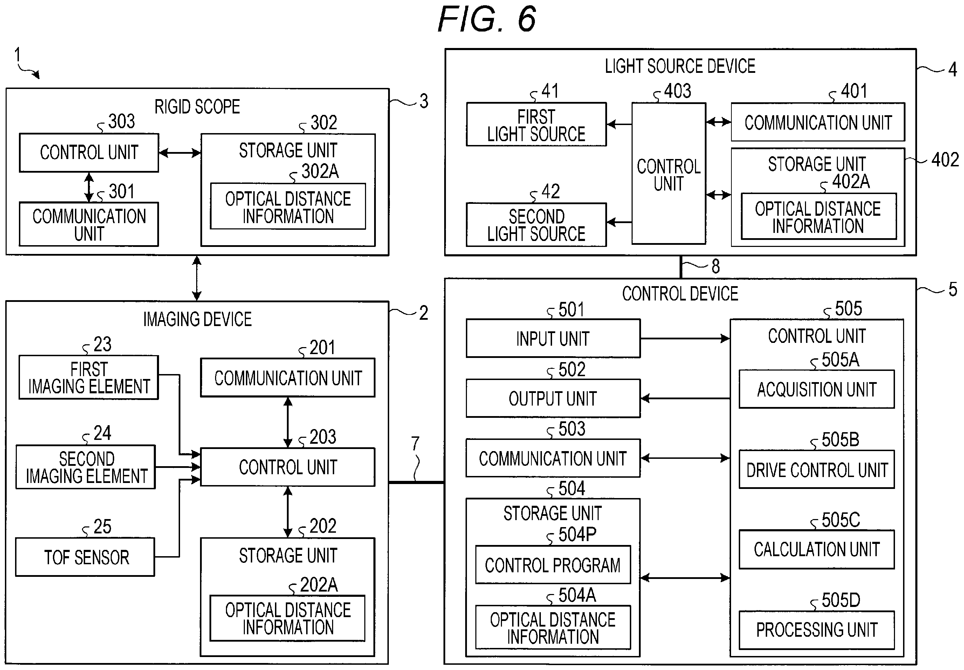

Next, a functional configuration of the endoscope system 1 according to the first embodiment will be described. Fig. 6 is a diagram showing an example of a functional configuration of the endoscope system 1 according to the first embodiment.

[Rigid scope]

As shown in Fig. 6, the rigid scope 3 includes a communication unit 301, a storage unit 302, and a control unit 303. The control unit 303 is electrically connected to the communication unit 301 and the storage unit 302.

The communication unit 301 communicates various types of information between with the imaging device 2 and the like. A communication protocol supported by the communication unit 301 is not particularly limited, and the communication unit 301 can also support a plurality of types of communication protocols.

The storage unit 302 stores various data and a program. For example, the storage unit 302 stores various types of information such as optical distance information 302A corresponding to a type of the rigid scope 3. The storage unit 302 is, for example, a semiconductor memory element or the like such as a RAM or a flash memory. The storage unit 302 stores the optical distance information 302A and the like. The optical distance information 302A includes, for example, information indicating the optical distance L3 of the illumination optical system 34 of the rigid scope 3, and the optical distance L4 of the imaging optical system 35. The storage unit 302 may store information for identifying the rigid scope 3.

The control unit 303 controls the communication unit 301 and the like. The control unit 303 is realized by, for example, an integrated circuit such as an application specific integrated circuit (ASIC) or a field-programmable gate array (FPGA). For example, when the rigid scope 3 is mounted to the imaging device 2, the control unit 303 transmits the optical distance information 302A to the imaging device 2 via the communication unit 301.

Note that, in the present embodiment, a case where the rigid scope 3 transmits the optical distance information 302A to the imaging device 2 is described, but the present invention is not limited to this. For example, the rigid scope 3 may have a configuration to transmit identification information to the imaging device 2, or may have a configuration to cause the imaging device 2 to read the identification information, the optical distance information 302A, and the like.

[Imaging device]

Next, an example of a functional configuration of the imaging device 2 will be described. As shown in Fig. 6, the imaging device 2 includes a communication unit 201, the storage unit 202, and the control unit 203. The control unit 203 is electrically connected to the communication unit 201 and the storage unit 202.

The communication unit 201 communicates various types of information between with the rigid scope 3, the control device 5, and the like. In the present embodiment, the communication unit 201 has a configuration to communicate with the control device 5 via the transmission cable 7. The communication unit 201 transmits information requested by the control unit 203, to the control device 5. The communication unit 201 outputs information received from the control device 5, to the control unit 203. Note that the communication unit 201 may have a configuration to perform wireless communication.

The storage unit 202 stores various data and a program. The storage unit 202 is, for example, a semiconductor memory element or the like such as a RAM or a flash memory. For example, the storage unit 202 stores various types of information such as optical distance information 202A and the like of the imaging device 2. The optical distance information 202A includes, for example, information indicating the optical distance L1 from the second light source 42 for the TOF sensor 25 of the imaging device 2, to the emission port 46. The storage unit 302 may store, for example, information for identifying the imaging device 2.

The control unit 203 controls the imaging device 2. The control unit 203 is realized by, for example, a CPU, a micro processing unit (MPU), or the like. The control unit 203 may be realized by, for example, an integrated circuit such as an ASIC or an FPGA. The control unit 203 is electrically connected to the first imaging element 23, the second imaging element 24, the TOF sensor 25, and the like. The control unit 203 has a function of outputting imaging information outputted from the first imaging element 23 and the second imaging element 24, to the control device 5. The control unit 203 has a function of outputting a detection result outputted from the TOF sensor 25, to the control device 5. For example, the control unit 203 has a function of transmitting, to the control device 5, the optical distance information 202A of the storage unit 202 and the optical distance information 302A received from the rigid scope 3, in response to a request or the like from the control device 5.

(Light source device)

Next, an example of a functional configuration of the light source device 4 will be described. As shown in Fig. 6, the light source device 4 includes the first light source 41, the second light source 42, a communication unit 401, a storage unit 402, and a control unit 403. The control unit 403 is electrically connected to the first light source 41, the second light source 42, the communication unit 401, and the storage unit 402.

The communication unit 401 communicates various types of information between with the control device 5 and the like. In the present embodiment, the communication unit 401 has a configuration to communicate with the control device 5 via the transmission cable 8. The communication unit 401 transmits information requested by the control unit 403, to the control device 5. The communication unit 401 outputs information received from the control device 5, to the control unit 403. Note that the communication unit 401 may have a configuration to perform wireless communication.

The storage unit 402 stores various data and a program. The storage unit 402 is, for example, a semiconductor memory element or the like such as a RAM or a flash memory. For example, the storage unit 402 stores optical distance information 402A and the like. The optical distance information 402A includes, for example, information indicating the optical distance L1 from the second light source 42 for the TOF sensor 25 to the emission port 46 in the light source device 4. The storage unit 402 may store information for identifying the light source device 4.

The control unit 403 controls the light source device 4. The control unit 403 is realized by, for example, a CPU, an MPU, or the like. The control unit 403 may be realized by, for example, an integrated circuit such as an ASIC or an FPGA. The control unit 403 is electrically connected to the first light source 41, the second light source 42, and the like. The control unit 403 has a function of controlling driving of the first light source 41, the second light source 42, and the like on the basis of a control signal or the like from the control device 5. The control unit 403 has a function of transmitting the optical distance information 402A of the storage unit 402 to the control device 5, for example, in response to a request or the like from the control device 5. The control unit 403 has, for example, a function of acquiring an optical distance, identification information, and the like of the light guide 6. In this case, the control unit 403 has a function of transmitting information regarding the light guide 6, to the control device 5.

Note that, in the present embodiment, a case where the light source device 4 transmits the optical distance information 402A to the control device 5 is described, but the present invention is not limited to this. For example, in a case where the light source device 4 and the control device 5 are used in combination, a configuration may be adopted in which the optical distance information 402A is stored in the control device 5 in advance.

[Control device]

Next, an example of a functional configuration of the control device 5 will be described. As shown in Fig. 6, the control device 5 includes an input unit 501, an output unit 502, a communication unit 503, a storage unit 504, and a control unit 505. The control unit 505 is electrically connected to the input unit 501, the output unit 502, the communication unit 503, and the storage unit 504.

The input unit 501 receives inputs of various types of information. The input unit 501 is realized by using, for example, a user interface such as a keyboard, a mouse, or a touch panel. The input unit 501 outputs received input information to the control unit 505.

The output unit 502 outputs various types of information. The output unit 502 is realized by using, for example, a display, a speaker, a printer, or the like. The output unit 502 outputs information requested by the control unit 505.

The communication unit 503 communicates various types of information between with the imaging device 2, the light source device 4, and the like. In the present embodiment, the communication unit 503 has a configuration to communicate with the imaging device 2 via the transmission cable 7. Then, the communication unit 503 has a configuration to communicate with the light source device 4 via the transmission cable 8. The communication unit 503 transmits information requested by the control unit 505, to the imaging device 2, the light source device 4, and the like. The communication unit 503 outputs information received from the imaging device 2, the light source device 4, and the like, to the control unit 505. Furthermore, the communication unit 503 has a configuration to be able to communicate various types of information between with the arm control device 5045 of the support arm 5027, by supporting a plurality of types of communication protocols. The communication unit 503 transmits information requested by the control unit 505, to the arm control device 5045. The communication unit 503 outputs information received from the arm control device 5045, to the control unit 505.

The storage unit 504 stores various data and a program. The storage unit 504 is also used as a work area for temporarily storing a processing result of the control unit 505. The storage unit 504 is, for example, a semiconductor memory element such as a RAM or a flash memory, a hard disk, an optical disk, or the like. Note that the storage unit 504 may be provided in a server connected to the control device 5 via the communication unit 503. For example, the storage unit 504 stores a control program 504P, optical distance information 504A, and the like. The control program 504P provides, for example, a function of calculating the distance D6 or the like from the tip end 31a of the rigid scope 3 to the subject M. The control program 504P provides, for example, a function of controlling an operation of the imaging device 2 and the light source device 4. The optical distance information 504A includes, for example, information indicating the optical distance L2 of the light guide 6. For example, the optical distance information 504A may be set by a user or the like, or may be read from the light guide 6.

The control unit 505 controls the imaging device 2, the light source device 4, the control device 5, and the like. The control unit 505 is realized by, for example, a CPU, an MPU, or the like. The control unit 505 may be realized by, for example, an integrated circuit such as an ASIC or an FPGA. The control unit 505 executes the control program 504P to control the imaging device 2, the light source device 4, the control device 5, and the like.

The control unit 505 includes an acquisition unit 505A, a drive control unit 505B, a calculation unit 505C, and a processing unit 505D. Each functional unit of the acquisition unit 505A, the drive control unit 505B, the calculation unit 505C, and the processing unit 505D is realized by the control unit 505 executing the control program 504P.

The acquisition unit 505A acquires each optical distance of the illumination optical system 34 and the imaging optical system 35 of the rigid scope 3, the light guide 6, the light source device 4, and the imaging device 2. In the present embodiment, for example, the acquisition unit 505A acquires each optical distance from the optical distance information 202A of the imaging device 2, the optical distance information 302A of the rigid scope 3, and the optical distance information 402A of the light source device 4. Then, the acquisition unit 505A acquires the optical distance of the light guide 6 from the storage unit 504, but may have a configuration to acquire the optical distance directly or indirectly from the light guide 6.

The drive control unit 505B has a function of controlling driving of the light source device 4. For example, in the endoscope system 1 according to the first embodiment, since the optical distance is different between the light emitting side and the light receiving side, a timing needs to be synchronized between the light emitting side and the light receiving side. Therefore, the drive control unit 505B has a function of synchronizing a light emission timing of the light source device 4 and the TOF sensor 25 via the communication unit 503. For example, the drive control unit 505B can perform synchronization by instructing light emission of the second light source 42 and instructing an imaging timing according to the light emission. Furthermore, the drive control unit 505B has a function of causing the first light source 41 and the second light source 42 of the light source device 4 to emit light at different timings.

The calculation unit 505C calculates the distance D6 from the tip end 31a of the rigid scope 3 to the subject M, on the basis of an optical distance acquired by the acquisition unit 505A and a detection result (flight time) of the TOF sensor 25. For example, the calculation unit 505C calculates the distance D6 from the tip end 31a of the rigid scope 3 to the subject M, by using the optical distance L1, the optical distance L2, the optical distance L3, the optical distance L4, the optical distance L5, the detection result of the TOF sensor 25, and Formula (1) described above.

The processing unit 505D executes processing of determining whether or not the distance D6 from the tip end 31a of the rigid scope 3 to the subject M calculated by the calculation unit 505C has become closer than a threshold value. In a case where the calculated distance to the subject M has become closer than the threshold value, the processing unit 505D executes processing for notifying that the rigid scope 3 is approaching the subject M. For example, the processing unit 505D transmits notification information for notifying that the rigid scope 3 is approaching the subject M, to the arm control device 5045 or the like via the communication unit 503. The notification information may include, for example, a distance from the tip end 31a of the rigid scope 3 to the subject M. As a result, the arm control device 5045 and the like can perform control to change a location, a position, and the like of the imaging device 2 of the endoscope system 1, on the basis of the received notification information. Furthermore, the processing unit 505D may execute a notification process regarding the calculated distance to the subject M. By notifying the distance to the subject M, the processing unit 505D can cause the arm control device 5045, an observer, and the like to execute control regarding the location, the position, and the like of the imaging device 2, on the basis of the distance from the tip end 31a of the rigid scope 3 to the subject M.

The control unit 505 may have, for example, an image processing function. The control unit 505 performs various types of image processing on an image signal transmitted from the imaging device 2. The image processing includes various types of known signal processing such as, for example, development processing, high image quality processing (such as band emphasizing processing, super resolution processing, noise reduction (NR) processing, and/or camera shake correction processing), enlargement processing (electronic zoom processing), and/or the like. Furthermore, the control unit 505 performs wave-detection processing on an image signal for performing AE, AF, and AWB.

The functional configuration example of the endoscope system 1 according to the present embodiment has been described above. Note that the configuration described above with reference to Fig. 6 is merely an example, and the functional configuration of the endoscope system 1 according to the present embodiment is not limited to the example. The functional configuration of the endoscope system 1 according to the present embodiment can be flexibly modified in accordance with specifications and operations.

[Processing procedure of control device according to first embodiment]

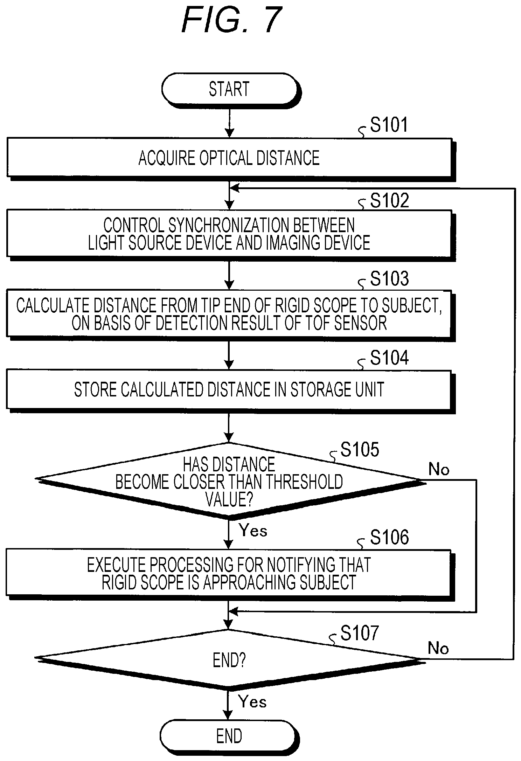

Next, an example of a processing procedure of the control device 5 according to the first embodiment will be described with reference to Fig. 7. Fig. 7 is a flowchart showing an example of a processing procedure executed by the control device 5 according to the first embodiment. The processing procedure shown in Fig. 7 is realized by the control unit 505 of the control device 5 executing the control program 504P.

As shown in Fig. 7, the control unit 505 of the control device 5 acquires an optical distance (step S101). For example, the control unit 505 collects the optical distance information 302A of the rigid scope 3, the optical distance information 202A of the imaging device 2, and the optical distance information 402A of the light source device 4 via the communication unit 503, and acquires and stores the optical distance L1, the optical distance L2, the optical distance L3, the optical distance L4, and the optical distance L5 shown in Fig. 5, in the storage unit 504. The control unit 505 functions as the acquisition unit 505A by executing the process of step S101.

The control unit 505 controls synchronization between the light source device 4 and the imaging device 2 (step S102). For example, the control unit 505 performs control to cause the first light source 41 and the second light source 42 of the light source device 4 to emit light at different timings. Then, when the control unit 505 causes, via the communication unit 503, the second light source 42 of the light source device 4 to emit light, the control unit 505 controls the synchronization by notifying the TOF sensor 25 of the light emission timing. The control unit 505 functions as the drive control unit 505B by executing the process of step S102.

The control unit 505 calculates the distance D6 from the tip end 31a of the rigid scope 3 to the subject M, on the basis of a detection result of the TOF sensor 25 (step S103). For example, the control unit 505 substitutes an optical distance and a time T, which is the detection result of the TOF sensor 25 into the Formula (1) described above, to calculate the distance D6. The control unit 505 functions as the calculation unit 505C by executing the process of step S103. Then, the control unit 505 stores the calculated distance D6 in the storage unit 504 (step S104).

The control unit 505 determines whether or not the distance D6 has become closer than a threshold value (step S105). In a case where the distance D6 is smaller than a preset threshold value, the control unit 505 determines that the vehicle has approached the subject M. As the threshold value, for example, a distance for notifying that the tip end 31a of the rigid scope 3 has approached the subject M is set. In a case where the control unit 505 determines that the distance D6 has not become closer than the threshold value (No in step S105), the process proceeds to step S107 described later. Furthermore, in a case where the control unit 505 determines that the distance D6 has become closer than the threshold value (Yes in step S105), the process proceeds to step S106.

The control unit 505 executes processing for notifying that the rigid scope 3 is approaching the subject M (step S106). For example, the control unit 505 executes processing of transmitting notification information for notifying that the rigid scope 3 is approaching the subject M, to the arm control device 5045 of the support arm 5027 via the communication unit 503. The control unit 505 functions as the processing unit 505D by executing the process of step S106. As a result, the arm control device 5045 of the support arm 5027 controls the support arm 5027 so as to change a location of the imaging device 2 and move the imaging device 2 away from the subject M. Then, when the process of step S106 ends, the control unit 505 advances the process to step S107.

The control unit 505 determines whether or not to end (step S107). For example, in a case where an end request is received from the arm control device 5045 or the like, the control unit 505 determines to end. In a case where the control unit 505 determines not to end (No in step S107), the process returns to step S102 already described, and the processing procedure from step S102 is continued. Furthermore, in a case where it is determined to end (Yes in step S107), the control unit 505 ends the processing procedure shown in Fig. 7.

As described above, in the endoscope system 1 according to the present embodiment, in a case where the distance D6 to the subject M has become closer than the threshold value, the control device 5 executes processing for notifying that the rigid scope 3 is approaching the subject M. With this configuration, the endoscope system 1 can cause the support arm 5027 to recognize that the rigid scope 3 is approaching the subject M, before coming into contact with the subject M. As a result, the endoscope system 1 can prevent the rigid scope 3 from colliding with the subject M, so that safety can be improved.

Furthermore, in the endoscope system 1, the control device 5 causes the first light source 41 and the second light source 42 to emit light at different timings. With this configuration, in the endoscope system 1, even in a case where wavelengths of the first light source 41 and the second light source 42 are close to each other, it is possible to cause the imaging device 2 to receive light in accordance with the light emission timing, for example, by the drive control unit 505B controlling to alternately emit light. As a result, the endoscope system 1 can avoid erroneous recognition of the imaging device 2 due to infrared light having a close wavelength.

Furthermore, in the endoscope system 1, the control device 5 includes the communication unit 503 that communicates with the light source device 4 and the imaging device 2, and synchronizes a timing at which the TOF sensor 25 and the second light source 42 emit light, via the communication unit 503. With this configuration, in the endoscope system 1, even when the light source device 4 includes the first light source 41 and the second light source 42, it is possible to suppress a decrease in detection accuracy of the TOF sensor 25. As a result, in the endoscope system 1, it is possible to improve the accuracy of the distance D6 to the subject M detected using the TOF sensor 25.

Furthermore, by the acquisition unit 505A of the control device 5, the endoscope system 1 acquires each optical distance of the illumination optical system 34 and the imaging optical system 35 of the rigid scope 3, the light guide 6, the light source device 4, and the imaging device 2. Then, the calculation unit 505C calculates a distance from the tip end 31a of the rigid scope 3 to the subject M, on the basis of the optical distance and a detection result of the TOF sensor 25. With this configuration, even when the rigid scope 3 mounted to the imaging device 2 is changed, the endoscope system 1 can acquire the optical distance and calculate the distance D6 from the tip end 31a of the mounted rigid scope 3 to the subject M. As a result, since the endoscope system 1 can use a wide variety of the rigid scopes 3, a commercial value can be improved.

[Modification of first embodiment]

For example, the control device 5 of the endoscope system 1 according to the first embodiment may have a configuration not to acquire the optical distance from at least one of the imaging device 2, the rigid scope 3, the light source device 4, or the light guide 6.

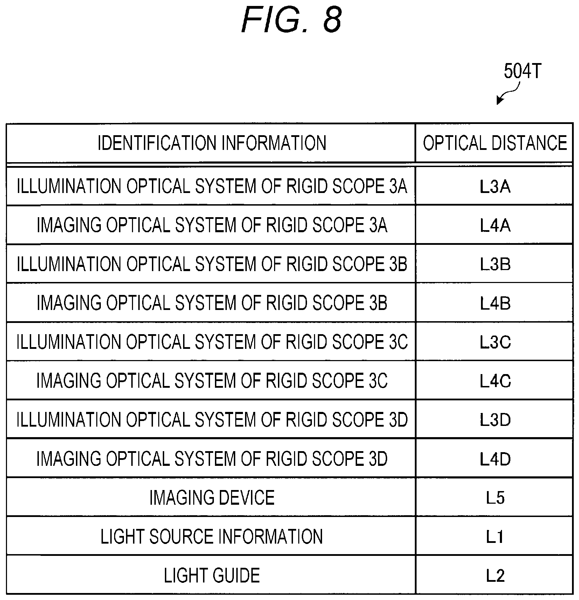

Fig. 8 is a view showing an example of optical distance information 504T of a control device 5 according to a modification of the first embodiment. The control device 5 of the endoscope system 1 may store the optical distance information 504T shown in Fig. 8 in the storage unit 504. In this case, the control device 5 is only required to have a configuration to acquire information to be identified by the imaging device 2, the rigid scope 3, the light source device 4, and the light guide 6. As an acquisition method, for example, the identification information may be acquired by communication, or may be acquired by causing a user or the like to input the identification information.

The optical distance information 504T is, for example, a table that associates identification information with an optical distance. In the example shown in Fig. 8, the optical distance information 504T associates an optical distance L3A in a case where the identification information is for the illumination optical system 34 of the rigid scope 3A, and associates an optical distance L4A in a case where the identification information is for the imaging optical system 35. Then, in a case where the identification information is for the imaging device 2, the optical distance information 504T associates the optical distance L5.

The acquisition unit 505A of the control device 5 is only required to change so as to acquire the optical distance from the optical distance information 504T, for example, on the basis of identification information of each of the imaging device 2, the rigid scope 3, the light source device 4, and the light guide 6. Furthermore, in a case where the acquisition unit 505A cannot acquire the identification information, the acquisition unit 505A may also acquire the optical distance from the imaging device 2, the rigid scope 3, the light source device 4, and the light guide 6, as described above. The acquisition unit 505A may combine acquisition methods.

As described above, in the endoscope system 1 according to the modification of the first embodiment, the control device 5 stores, in the storage unit 504, the optical distance information 504T of the rigid scope 3 and the light guide 6 that may be mounted. In the endoscope system 1, the control device 5 acquires the optical distance from the optical distance information 504T on the basis of the identification information. With this configuration, the endoscope system 1 does not need to store the optical distance in the imaging device 2, the rigid scope 3, the light source device 4, and the light guide 6. As a result, the endoscope system 1 can suppress complexity of the system by suppressing a storage capacity of the imaging device 2, the rigid scope 3, the light source device 4, and the light guide 6.

Note that the modification of the first embodiment may be applied to the endoscope system 1 of other embodiments and modifications.

(Second embodiment)

[Configuration example of endoscope system according to second embodiment]

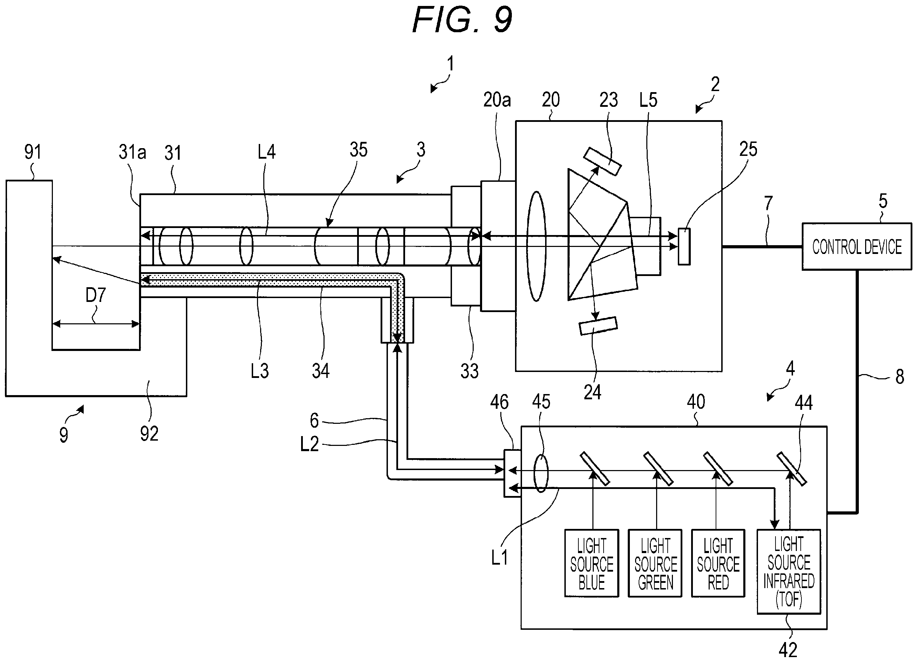

Next, a second embodiment will be described. Fig. 9 is a view showing an example of a configuration of an endoscope system 1 according to a second embodiment. For example, there is a possibility that the endoscope system 1 uses an existing rigid scope 3, light guide 6, and the like. In this case, the existing rigid scope 3, light guide 6, and the like may not have an optical distance or identification information. Furthermore, there is a possibility that the endoscope system 1 does not have an optical distance or identification information also in a case of using a rigid scope 3, a light guide 6, or the like of another manufacturer or the like. Therefore, the endoscope system 1 according to the second embodiment can use the rigid scope 3, the light guide 6, and the like that do not have an optical distance or identification information.

As shown in Fig. 9, the endoscope system 1 includes an imaging device 2, a rigid scope 3, a light source device 4, a control device 5, a light guide 6, and a jig 9. The jig 9 is used, for example, at a time of initial setting, maintenance, or the like of the endoscope system 1, and is not used in a case of capturing an image of a subject M. That is, the jig 9 may not be included in a configuration of the endoscope system 1.

For example, in a case where the endoscope system 1 uses the rigid scope 3 and the light guide 6 that do not have the optical distance information, it is assumed that only an optical distance L1 of the light source device 4 on a light emitting side and the optical distance L5 of the imaging device 2 on a light receiving side are known in advance. In this case, the endoscope system 1 cannot calculate absolute values of an optical distance of the light guide 6 and an optical distance of the rigid scope 3. However, if a distance D6 is known in advance, the sum of the optical distances of the rigid scope 3 and the light guide 6 can be obtained. Then, if the sum of the optical distances of the rigid scope 3 and the light guide 6 can be obtained, the endoscope system 1 can obtain the sum of optical distances (L1 + L2 + L3 + L4 + L5).

The jig 9 is a jig that causes reflect light emitted from a tip end 31a of the rigid scope 3, to be reflected to the tip end 31a at a predetermined location. The jig 9 is a jig to position (fix) a reflection unit 19 at a location away from the tip end 31a of the rigid scope 3 by a predetermined distance. For example, the predetermined distance is a preset reference distance D7, and is stored in optical distance information 504A, a control program 504P, and the like of a storage unit 504 of the control device 5.

The jig 9 includes a reflection unit 91 and a positioning unit 92. The reflection unit 91 is a reflecting member facing the tip end 31a of the rigid scope 3. The reflection unit 91 reflects infrared light emitted from the tip end 31a of the rigid scope 3, toward the tip end 31a. The reflection unit 91 is an example of a target object. The positioning unit 92 is a member that positions, by being mounted to the rigid scope 3, a reflecting surface of the reflection unit 91 at a location separated from the tip end 31a of the rigid scope 3 by the reference distance D7. The positioning unit 92 is formed by, for example, synthetic resin or the like.

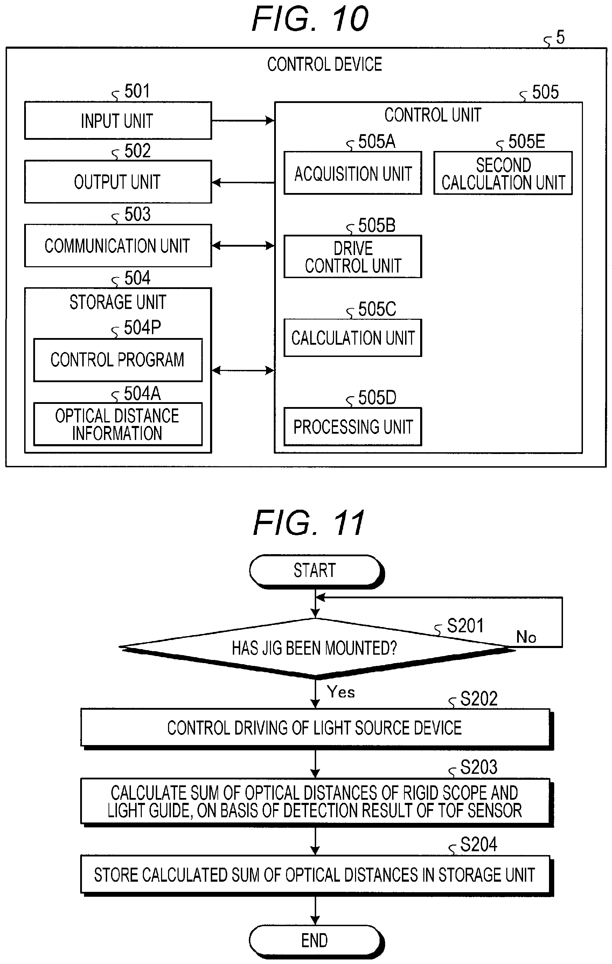

Fig. 10 is a diagram showing a configuration of the control device 5 according to the second embodiment. As shown in Fig. 10, the control device 5 includes an acquisition unit 505A, a drive control unit 505B, a calculation unit 505C, a processing unit 505D, and a second calculation unit 505E. Each functional unit of the acquisition unit 505A, the drive control unit 505B, the calculation unit 505C, the processing unit 505D, and the second calculation unit 505E is realized by the control unit 505 executing the control program 504P.

The second calculation unit 505E calculates the sum of the optical distances of the rigid scope 3 and the light guide 6, on the basis of a detection result of the TOF sensor 25 using the reflection unit 91 of the jig 9, the reference distance (predetermined distance) D7, and optical distances of the light source device 4 and the imaging device 2. For example, when the jig 9 is mounted and the reflection unit 91 faces the tip end 31a of the rigid scope 3, the control unit 505 instructs the drive control unit 505B to drive the light source device 4. Then, the second calculation unit 505E calculates the sum (L2 + L3 + L4) of the optical distances of the rigid scope 3 and the light guide 6, on the basis of the detection result of the TOF sensor 25, the reference distance D7, and the optical distances L1 and L5 of the light source device 4 and the imaging device 2. In other words, the second calculation unit 505E calculates the sum (L2 + L3 + L4) of the illumination optical system 34 and the imaging optical system 35 of the rigid scope 3 and the optical system of the light guide 6.

For example, it is assumed that a speed of light is V, and a time from when the light source device 4 emits light to when the light is received by the TOF sensor 25 is T. In this case, the sum (L2 + L3 + L4) of the optical distances of the rigid scope 3 and the light guide 6 can be calculated by the following Formula (2).

$$L2 + L3 + L4 = [(V \times T) - (L1 + L5 + D7 \times 2)] \cdots$$

Formula (2)

The acquisition unit 505A acquires the sum of the optical distances calculated by the second calculation unit 505E and the optical distances of the light source device 4 and the imaging device 2. Then, the calculation unit 505C calculates the distance D6 from the tip end 31a of the rigid scope 3 to the subject M on the basis of the optical distance and the sum of the optical distances acquired by the acquisition unit 505A, and a detection result of the TOF sensor 25.

The functional configuration example of the endoscope system 1 according to the second embodiment has been described above. Note that the functional configuration described above with reference to Fig. 10 is merely an example, and the functional configuration of the endoscope system 1 according to the second embodiment is not limited to the example. The functional configuration of the endoscope system 1 according to the second embodiment can be flexibly modified in accordance with specifications and operations.

[Processing procedure of control device according to second embodiment]

Next, a setting example using the jig 9 of the control device 5 according to the second embodiment will be described with reference to Fig. 11. Fig. 11 is a flowchart showing an example of a setting process executed by the control device 5 according to the second embodiment. The setting process shown in Fig. 11 is realized by the control unit 505 of the control device 5 executing the control program 504P. Note that, as a premise, the optical distances L1 and L5 of the light source device 4 and the imaging device 2 and the reference distance D7 of the jig 9 are stored in the optical distance information 504A of the storage unit 504.

As shown in Fig. 11, the control unit 505 of the control device 5 determines whether or not the jig 9 has been mounted (step S201). For example, in a case where it is detected that individual contact points or the like of the jig 9 and the rigid scope 3 are in electrical contact with each other, that a setting request is received from a user, or the like, the control unit 505 determines that the jig 9 has been mounted. In a case where it is determined that the jig 9 has not been mounted (No in step S201), the control unit 505 repeats this determination process to wait for the jig 9 to be mounted. Furthermore, in a case where it is determined that the jig 9 has been mounted (Yes in step S201), the control unit 505 advances the process to step S202.

The control unit 505 controls driving of the light source device 4 (step S202). For example, when the control unit 505 causes, via the communication unit 503, the second light source 42 of the light source device 4 to emit light, the control unit 505 notifies the TOF sensor 25 at the timing of the light emission. Then, the control unit 505 calculates the sum of optical distances of the rigid scope 3 and the light guide 6, on the basis of a detection result of the TOF sensor 25 (step S203). For example, the control unit 505 substitutes the detection result of the TOF sensor 25, the reference distance D7, and the optical distances L1 and L5 of the light source device 4 and the imaging device 2 into Formula (2) described above, to calculate the sum (L2 + L3 + L4) of the optical distances of the rigid scope 3 and the light guide 6.

The control unit 505 stores the calculated sum of the optical distances in the storage unit 504 (step S204). When the process of step S204 ends, the control unit 505 ends the processing procedure shown in Fig. 11.

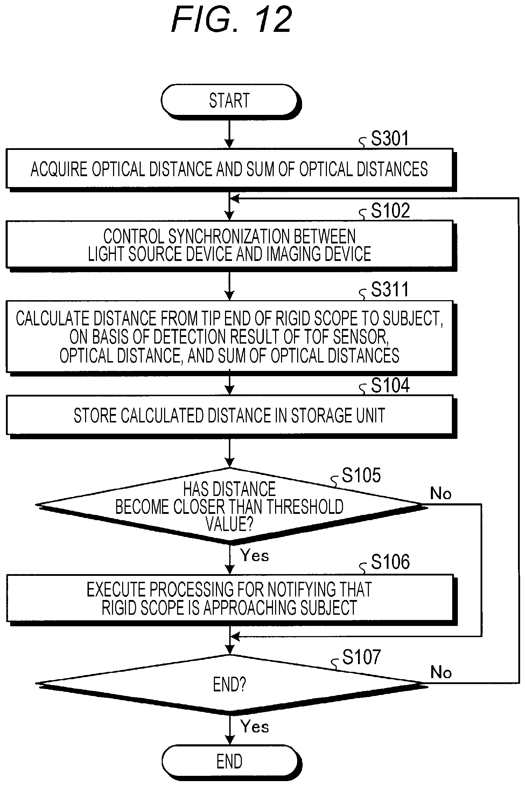

Next, an example of a processing procedure of the control device 5 according to the second embodiment will be described with reference to Fig. 12. Fig. 12 is a flowchart showing an example of a processing procedure executed by the control device 5 according to the second embodiment. The processing procedure shown in Fig. 12 is realized by the control unit 505 of the control device 5 executing the control program 504P. The processing procedure shown in Fig. 12 is executed in a state where the sum (L2 + L3 + L4) of the optical distances of the rigid scope 3 and the light guide 6 is stored in the storage unit 504 of the control device 5.

As shown in Fig. 12, the control unit 505 of an information processing apparatus 10 acquires the optical distance and the sum of the optical distances (step S301). For example, the control unit 505 acquires the sum of the optical distances of the rigid scope 3 and the light guide 6 from the storage unit 504. As described above, the control unit 505 acquires the optical distances from the optical distance information 202A of the imaging device 2 and the optical distance information 402A of the light source device 4. The control unit 505 functions as the acquisition unit 505A by executing the process of step S301.

The control unit 505 controls synchronization between the light source device 4 and the imaging device 2 (step S102). Then, on the basis of a detection result of the TOF sensor 25 and the acquired optical distance and sum of the optical distances, the control unit 505 calculates the distance D6 from the tip end 31a of the rigid scope 3 to the subject M (step S311). For example, the control unit 505 substitutes the acquired optical distance and sum of the optical distances and a time T that is the detection result of the TOF sensor 25 into Formula (1) described above, to calculate the distance D6. The control unit 505 functions as the calculation unit 505C by executing the process of step S311.

Steps 104 to S107 shown in Fig. 12 are the same as steps S104 to S107 shown in Fig. 7 described above. The control unit 505 stores the calculated distance D6 in the storage unit 504 (step S104). The control unit 505 determines whether or not the distance D6 has become closer than a threshold value (step S105). In a case where the control unit 505 determines that the distance D6 has not become closer than the threshold value (No in step S105), the process proceeds to step S107 described later. Furthermore, in a case where the control unit 505 determines that the distance D6 has become closer than the threshold value (Yes in step S105), the process proceeds to step S106.

The control unit 505 executes processing for notifying that the rigid scope 3 is approaching the subject M (step S106). As a result, the arm control device 5045 of the support arm 5027 controls the support arm 5027 so as to change a location of the imaging device 2 and move the imaging device 2 away from the subject M.

The control unit 505 determines whether or not to end (step S107). In a case where the control unit 505 determines not to end (No in step S107), the process returns to step S102 already described, and the processing procedure from step S102 is continued. Furthermore, in a case where it is determined to end (Yes in step S107), the control unit 505 ends the processing procedure shown in Fig. 12.

As described above, the endoscope system 1 according to the present embodiment further includes the jig 9 that positions the reflection unit 91 at a location at a predetermined distance from the tip end 31a of the rigid scope 3. In the endoscope system 1, the second calculation unit 505E of the control device 5 calculates the sum of the optical distances of the rigid scope 3 and the light guide 6, on the basis of a detection result of the TOF sensor 25 using the reflection unit 91 of the jig 9, the predetermined distance, and the optical distances of the light source device 4 and the imaging device 2. Then, the endoscope system 1 acquires the optical distances of the rigid scope 3 and the light guide 6 on the basis of the sum of the optical distances calculated by the second calculation unit 505E. With this configuration, in the endoscope system 1, even if the rigid scope 3 or the light guide 6 is changed, the distance D6 from the tip end 31a of the rigid scope 3 to the subject M can be calculated on the basis of the detection result of the TOF sensor 25. As a result, since the endoscope system 1 can use a wide variety of the rigid scopes 3 and the light guides 6, it is possible to further improve the commercial value.

[Modification of second embodiment]

For example, even if the imaging device 2, the light source device 4, the light guide 6, and the like have information indicating an optical distance, there is a possibility that an error occurs between an actual optical distance and the held optical distance due to aging or the like. An endoscope system 1 according to a modification of the second embodiment is an example of a case where the jig 9 described above is used as a calibration jig.

The control device 5 corrects a difference between the reference distance D7 of the jig 9 and the actually detected distance D6, by detecting the distance D6 by the TOF sensor 25 in a state where the jig 9 is mounted. As a result, the control device 5 can calculate the distance D6 with high accuracy by calibration using the jig 9.

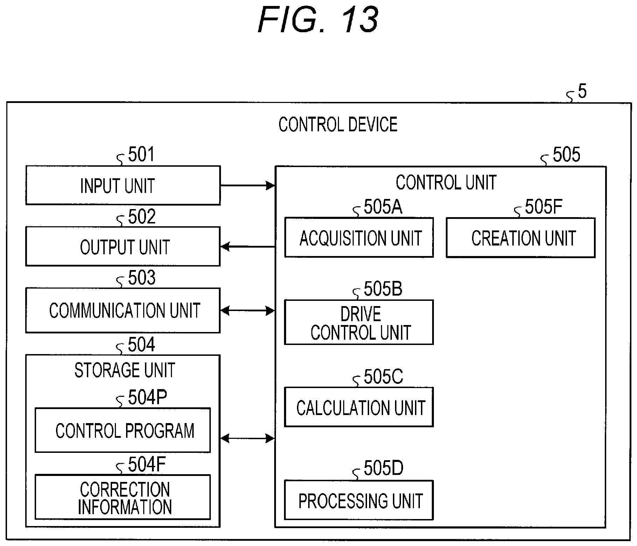

Fig. 13 is a view showing a configuration of a control device 5 according to a modification of the second embodiment. As shown in Fig. 13, the control device 5 includes an acquisition unit 505A, a drive control unit 505B, a calculation unit 505C, a processing unit 505D, and a creation unit 505F. Each functional unit of the acquisition unit 505A, the drive control unit 505B, the calculation unit 505C, the processing unit 505D, and the creation unit 505F is realized by the control unit 505 executing the control program 504P.

In a case where the reflection unit 91 is positioned at a location at the reference distance (predetermined distance) D7 from the tip end 31a of the rigid scope 3 by the jig 9, the creation unit 505F creates correction information 504F on the basis of the distance D6 calculated by the calculation unit 505C and the reference distance D7.

For example, it is assumed that the endoscope system 1 stores in advance the optical distances L1, L2, L3, L4, and L5, which are design values. In this case, due to aging or the like, actual values of the optical distances L1, L2, L3, L4, and L5 may be changed to optical distances L1', L2', L3', L4', and L5'. The sum of the actual optical distances can be obtained by the following Formula (3), by mounting the jig 9 described above and detecting a time from light emission of the light source device 4 to light reception of the TOF sensor 25. Note that, it is assumed that a speed of light is V, and a time from when the light source device 4 emits light to when the light is received by the TOF sensor 25 is T.

$$\text{L1'} + \text{L2'} + \text{L3'} + \text{L4'} + \text{L5'} = [V \times T - D7 \times 2] \cdots$$

Formula (3)

By obtaining L1' + L2' + L3' + L4' + L5', the endoscope system 1 can calculate the actual distance D6 by using Formula (1) described above. Therefore, in the present embodiment, the creation unit 505F creates the correction information 504F including the sum of the optical distances of L1' + L2' + L3' + L4' + L5', and stores the correction information 504F in the storage unit 504. The correction information 504F in the storage unit 504 may be periodically updated, for example.

The acquisition unit 505A acquires the correction information 504F created by the creation unit 505F. For example, the acquisition unit 505A acquires the correction information 504F in a case where the correction information 504F is stored in the storage unit 504, and acquires the optical distance in a case where the correction information 504F is not stored in the storage unit 504. Then, the calculation unit 505C calculates the distance D6 from the tip end 31a of the rigid scope 3 to the subject M on the basis of the correction information 504F acquired by the acquisition unit 505A and a detection result of the TOF sensor 25.

[Processing procedure of control device according to modification of second embodiment]

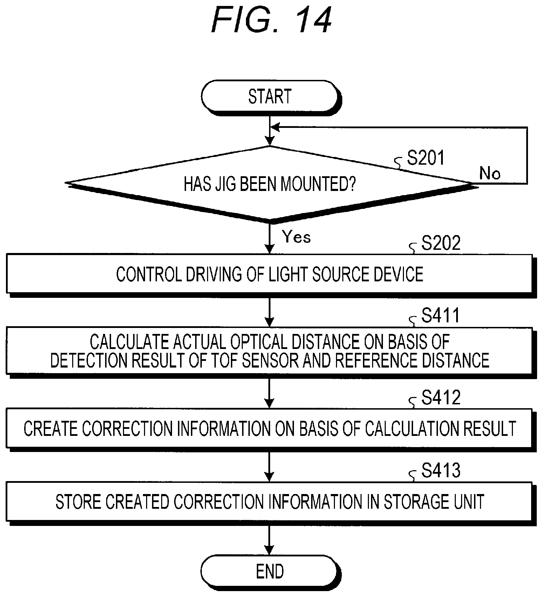

Next, a setting example using the jig 9 of the control device 5 according to a modification of the second embodiment will be described with reference to Fig. 14. Fig. 14 is a flowchart showing an example of a setting process executed by the control device 5 according to a modification of the second embodiment. The setting process shown in Fig. 14 is realized by the control unit 505 of the control device 5 executing the control program 504P.

As shown in Fig. 14, the control unit 505 of the control device 5 determines whether or not the jig 9 has been mounted (step S201). In a case where it is determined that the jig 9 has not been mounted (No in step S201), the control unit 505 repeats this determination process to wait for the jig 9 to be mounted. Furthermore, in a case where it is determined that the jig 9 has been mounted (Yes in step S201), the control unit 505 advances the process to step S202.

The control unit 505 controls driving of the light source device 4 (step S202). Then, the control unit 505 calculates an actual optical distance, on the basis of a detection result of the TOF sensor 25 and the reference distance D7 (step S411). For example, the control unit 505 substitutes the detection result (flight time) of the TOF sensor 25 and the reference distance D7 into Formula (3) described above, to calculate the sum of the actual optical distances (L1' + L2' + L3' + L4' + L5').

The control unit 505 creates correction information on the basis of the calculation result (step S412). For example, the control unit 505 creates the correction information 504F including the sum of the optical distances of L1' + L2' + L3' + L4' + L5'. Then, the control unit 505 stores the created correction information 504F in the storage unit 504 (step S413). When the process of step S413 ends, the control unit 505 ends the processing procedure shown in Fig. 14.

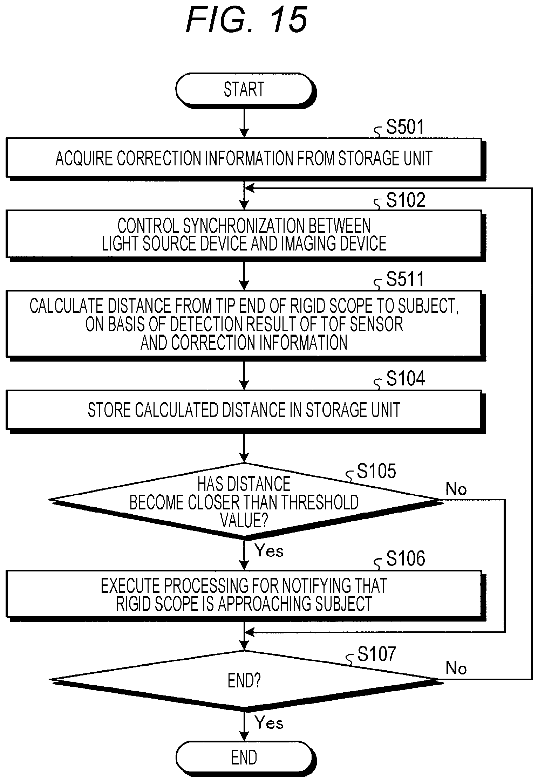

Next, an example of a processing procedure of the control device 5 according to a modification of the second embodiment will be described with reference to Fig. 15. Fig. 15 is a flowchart showing an example of a processing procedure executed by the control device 5 according to the modification of the second embodiment. The processing procedure shown in Fig. 15 is realized by the control unit 505 of the control device 5 executing the control program 504P. The processing procedure shown in Fig. 15 is executed in a state where the correction information 504F is stored in the storage unit 504 of the control device 5.

As shown in Fig. 15, the control unit 505 of the information processing apparatus 10 acquires the correction information 504F from the storage unit 504 (step S501). For example, the control unit 505 acquires the correction information 504F including the sum of the optical distances of L1' + L2' + L3' + L4' + L5' from the storage unit 504. The control unit 505 functions as the acquisition unit 505A by executing the process of step S501.

The control unit 505 controls synchronization between the light source device 4 and the imaging device 2 (step S102). Then, the control unit 505 calculates the distance D6 from the tip end 31a of the rigid scope 3 to the subject M, on the basis of a detection result of the TOF sensor 25 and the correction information 504F (step S511). For example, the control unit 505 substitutes the sum of the optical distances indicated by the acquired correction information 504F and a time T that is the detection result of the TOF sensor 25 into Formula (1) described above, to calculate the distance D6. The control unit 505 functions as the calculation unit 505C by executing the process of step S511.

Steps S104 to S107 shown in Fig. 15 are the same as steps S104 to S107 shown in Fig. 7 described above. The control unit 505 stores the calculated distance D6 in the storage unit 504 (step S104). The control unit 505 determines whether or not the distance D6 has become closer than a threshold value (step S105). In a case where the control unit 505 determines that the distance D6 has not become closer than the threshold value (No in step S105), the process proceeds to step S107 described later. Furthermore, in a case where the control unit 505 determines that the distance D6 has become closer than the threshold value (Yes in step S105), the process proceeds to step S106.

The control unit 505 executes processing for notifying that the rigid scope 3 is approaching the subject M (step S106). The control unit 505 determines whether or not to end (step S107). In a case where the control unit 505 determines not to end (No in step S107), the process returns to step S102 already described, and the processing procedure from step S102 is continued. Furthermore, in a case where it is determined to end (Yes in step S107), the control unit 505 ends the processing procedure shown in Fig. 15.

As described above, in the endoscope system 1 according to the present embodiment, in a case where the reflection unit 91 is positioned at a location at the reference distance (predetermined distance) D7 from the tip end 31a of the rigid scope 3 by the jig 9, the creation unit 505F creates the correction information 504F, on the basis of the distance D6 from the tip end 31a of the rigid scope 3 to the subject M calculated by the calculation unit 505C of the control device 5 and on the basis of and the reference distance D7. The endoscope system 1 acquires an optical distance included in an optical path on the basis of the correction information 504F created by the creation unit 505F. With this configuration, in a case where an error occurs between the optical distance stored in advance and an actual optical distance, the endoscope system 1 can adjust the optical distance by using the correction information 504F. As a result, even if an error occurs between the optical distance stored in advance and the actual optical distance, the endoscope system 1 can calculate the distance D6 with higher accuracy.

Note that the creation unit 505F of the modification of the second embodiment may create the correction information 504F for correcting the optical distances L1, L2, L3, L4, and L5 stored in advance. For example, the creation unit 505F may obtain a difference between the optical distance of the design value and the actual optical distance, and may create the correction information 504F for changing the optical distance of the design value in a case where the difference is equal to or larger than the threshold value.

The preferred embodiments of the present disclosure have been described above in detail with reference to the accompanying drawings, but the technical scope of the present disclosure is not limited to such examples. It is obvious that those with ordinary skill in the technical field of the present disclosure can arrive various variations or modifications within the scope of the technical idea described in the claims, and it is naturally understood that these also fall within the technical scope of the present disclosure.

Furthermore, the effects described in the present specification are merely exemplary or illustrative, and not restrictive. That is, the technology according to the present disclosure can exhibit other effects apparent to those skilled in the art from the description of the present specification, in addition to the effect described above or instead of the effect described above.

Furthermore, each step related to the processing of the information processing apparatus of the present specification is not necessarily processed in time series in the order described in the flowchart. For example, the individual steps related to the processing of the information processing apparatus may be processed in an order different from the order described in the flowchart, or may be processed in parallel.

Furthermore, the description has been given to a case where the endoscope system 1 of the present specification calculates a distance from the tip end 31a of the rigid scope 3 to the target object on the basis of the time of flight detected by the TOF sensor 25, but the present invention is not limited to this. For example, the endoscope system 1 may have a configuration to calculate the distance on the basis of a phase.

Note that, in the present specification, the endoscope system 1 has been described as an example of a medical observation system, but the present invention is not limited to this. For example, the medical observation system can also be applied to an exoscope system or the like including an exoscope. The exoscope is not inserted into the body, and is used, for example, for observing a subject from outside the body in a state of thoracotomy/laparotomy. That is, a lens barrel of the medical observation system includes an endoscope and an exoscope different from the rigid scope 3, in addition to the rigid scope 3 of the present specification. Then, the medical purpose observation system can be selectively mounted with a plurality of types of exoscopes by being applied to an exoscope system.

(Effect)

The endoscope system 1 includes: the imaging device 2 including the TOF sensor 25 capable of detecting a distance to a target object, and the first imaging element 23; the rigid scope 3 having the illumination optical system 34 and the imaging optical system 35 and replaceably mounted to the imaging device 2; and the light source device 4 provided outside the imaging device 2 and the rigid scope 3, and connected to the illumination optical system 34 of the rigid scope 3 via the light guide 6. The light source device 4 includes: the first light source 41 configured to emit light (first light) that is guided in the illumination optical system 34 and illuminates a target object; and the second light source 42 configured to emit infrared light (second light) that is guided in the illumination optical system 34 and to be detected by the TOF sensor 25. The TOF sensor 25 receives infrared light having been reflected by the target object and passed through the imaging optical system 35 of the rigid scope 3.

With this configuration, in the endoscope system 1, by providing the second light source 42 that emits infrared light for the TOF sensor 25 in the light source device 4 including the first light source 41 for illumination, it is not necessary to provide a light source for the TOF sensor 25 in the imaging device 2, and infrared light for the TOF sensor 25 can be emitted from the illumination optical system 34. As a result, in the endoscope system 1, when the target object is irradiated with the infrared light for the TOF sensor 25, it is not necessary to pass through the imaging optical system 35 of the rigid scope 3. Therefore, the loss of the infrared light due to the imaging optical system 35 can be suppressed. Furthermore, in the endoscope system 1, even when a plurality of types of the rigid scopes 3 is selectively mounted, a decrease in an amount of infrared light received by the TOF sensor 25 can be suppressed. Therefore, stability of the detection accuracy of the TOF sensor 25 can be improved.

The endoscope system 1 further includes the control device 5 configured to control the light source device 4. The control device 5 calculates a distance from the tip end 31a of the rigid scope 3 to the target object on the basis of a detection result of the TOF sensor 25.

With this configuration, the endoscope system 1 can calculate the distance from the tip end 31a of the mounted rigid scope 3 to the target object even when the rigid scope 3 having a different length, thickness, and the like is mounted to the imaging device 2, for example. As a result, in the endoscope system 1, a plurality of different types of the rigid scopes 3 can be mounted to the imaging device 2, which can improve versatility.

In the endoscope system 1, the rigid scope 3 guides infrared light from the second light source 42 by the illumination optical system 34, to emit toward the target object.

With this configuration, in the endoscope system 1, even if the length or the like of the rigid scope 3 is different, the illumination optical system 34 can be made common, or a length of the illumination optical system 34 can be made different. As a result, in the endoscope system 1, since a wide variety of the rigid scopes 3 can be replaced, convenience can be improved.

In the endoscope system 1, the control device 5 executes a notification process regarding a distance to the target object, on the basis of the calculated distance to the target object.

With this configuration, the endoscope system 1 can execute the notification process regarding the calculated distance to the target object. As a result, the endoscope system 1 can contribute to improvement of accuracy of location control and the like of the rigid scope 3, by the notification regarding the distance between the rigid scope 3 and the target object.

In the endoscope system 1, the control device 5 notifies of a distance to the target object.

With this configuration, the endoscope system 1 can notify of the distance of the rigid scope 3 to the target object. As a result, the endoscope system 1 can prevent the rigid scope 3 from excessively approaching the target object, by notifying of the distance between the rigid scope 3 and the target object.

In the endoscope system 1, in a case where the calculated distance to the target object becomes closer than a threshold value, the control device 5 executes processing for notifying that the rigid scope 3 is approaching the target object.

With this configuration, the endoscope system 1 can notify that the rigid scope 3 is approaching the target object, before coming into contact with the target object. As a result, the endoscope system 1 can prevent the rigid scope 3 from colliding with the target object, so that safety can be improved.

In the endoscope system 1, the control device 5 causes the first light source 41 and the second light source 42 to emit light at different timings.

With this configuration, in the endoscope system 1, even in a case where wavelengths of the first light source 41 and the second light source 42 are close to each other, it is possible to cause the imaging device 2 to receive light in accordance with the light emission timing, for example, by the drive control unit 505B controlling to alternately emit light. As a result, the endoscope system 1 can avoid erroneous recognition of the imaging device 2 due to infrared light having a close wavelength.

In the endoscope system 1, the control device 5 further includes the communication unit 503 that communicates with at least one of the light source device 4 or the imaging device 2. The control device 5 synchronizes a timing at which the TOF sensor 25 and the second light source 42 emit light, via the communication unit 503.

With this configuration, in the endoscope system 1, even when the light source device 4 includes the first light source 41 and the second light source 42, it is possible to suppress a decrease in detection accuracy of the TOF sensor 25. As a result, in the endoscope system 1, it is possible to improve the accuracy of the distance to the subject M detected using the TOF sensor 25.

In the endoscope system 1, the control device 5 further includes: the acquisition unit 505A that acquires each optical distance of the illumination optical system 34 and the imaging optical system 35 of the rigid scope 3, the light guide 6, the light source device 4, and the imaging device 2; and the calculation unit 505C that calculates a distance from the tip end 31a of the rigid scope 3 to the target object on the basis of an optical distance acquired by the acquisition unit 505A and a detection result of the TOF sensor 25.

With this configuration, even when the rigid scope 3 mounted to the imaging device 2 is changed, the endoscope system 1 can acquire the optical distance and calculate the distance from the tip end 31a of the mounted rigid scope 3 to the target object. As a result, since the endoscope system 1 can use a wide variety of the rigid scopes 3, a commercial value can be improved.

In the endoscope system 1, the control device 5 further includes the storage unit 504 that stores optical distance information indicating an optical distance of at least one of the rigid scope 3 or the light guide 6 of a plurality of types. The acquisition unit 505A acquires the optical distance of at least one of the rigid scope 3 or the light guide 6, from the optical distance information stored in the storage unit 504.

With this configuration, the endoscope system 1 does not need to store the optical distance in the imaging device 2, the rigid scope 3, the light source device 4, and the light guide 6. As a result, the endoscope system 1 can suppress complexity of the system by suppressing a storage capacity of the imaging device 2, the rigid scope 3, the light source device 4, and the light guide 6.

The endoscope system 1 further includes the jig 9 that positions the reflection unit 91 at a location at a predetermined distance from the tip end 31a of the rigid scope 3. The control device 5 further includes the second calculation unit 505E that calculates the sum of the optical distances of the rigid scope 3 and the light guide 6, on the basis of a detection result of the TOF sensor 25 using the reflection unit 91 of the jig 9, a predetermined distance, and optical distances of the light source device 4 and the imaging device 2. The acquisition unit 505A acquires the optical distance on the basis of the sum of the optical distances calculated by the second calculation unit 505E.

With this configuration, even when the rigid scope 3 or the light guide 6 is changed, the endoscope system 1 can calculate the distance from the tip end 31a of the rigid scope 3 to the target object, on the basis of the detection result of the TOF sensor 25. As a result, since the endoscope system 1 can use a wide variety of the rigid scopes 3 and the light guides 6, it is possible to further improve the commercial value.

In the endoscope system 1, the control device 5 further includes the creation unit 505F that creates the correction information 504F, on the basis of a distance to the target object from the tip end 31a of the rigid scope 3 calculated by the calculation unit 505C and on the basis of the predetermined distance, in a case where the reflection unit 91 is positioned at a location at a predetermined distance from the tip end 31a of the rigid scope 3 by the jig 9. The acquisition unit 505A acquires the optical distance on the basis of the correction information 504F created by the creation unit 505F.

With this configuration, in a case where an error occurs between the optical distance stored in advance and an actual optical distance, the endoscope system 1 can adjust the optical distance by using the correction information 504F. As a result, even if an error occurs between the optical distance stored in advance and the actual optical distance, the endoscope system 1 can calculate the distance with higher accuracy.

The medical system 5020 includes the endoscope system 1, and the support arm 5027 that has a plurality of links rotatably connected by a joint unit and is configured to be able to hold the imaging device 2 that observes the inside of the body. The endoscope system 1 includes: the imaging device 2 including the TOF sensor 25 capable of detecting a distance to a target object; the rigid scope 3 having the illumination optical system 34 and the imaging optical system 35 and replaceably mounted to the imaging device 2; and the light source device 4 provided outside the imaging device 2 and the rigid scope 3, and connected to the illumination optical system 34 of the rigid scope 3 via the light guide 6. The light source device 4 includes: the first light source 41 configured to emit visible light that is guided in the illumination optical system 34 and illuminates a target object; and the second light source 42 configured to emit infrared light that is guided in the illumination optical system 34 and to be detected by the TOF sensor 25. The TOF sensor 25 receives infrared light having been reflected by the target object and passed through the imaging optical system 35 of the rigid scope 3.

With this configuration, the medical system 5020 can move a location of the rigid scope 3 with high accuracy even when a plurality of types of the rigid scope 3 is used in the endoscope system 1. As a result, in the medical system 5020, since it is not necessary to pass through the imaging optical system 35 of the rigid scope 3 when the target object is irradiated with the infrared light for the TOF sensor 25 by the endoscope system 1, the loss of the infrared light due to the imaging optical system 35 can be suppressed. Furthermore, in the medical system 5020, since it is possible to suppress a decrease in an amount of infrared light received by the TOF sensor 25 even when a plurality of types of the rigid scope 3 is selectively mounted to the endoscope system 1, stability of the detection accuracy of the TOF sensor 25 can be improved.

The medical system 5020 further includes the arm control device 5045 configured to control the support arm 5027. The arm control device 5045 controls the joint units 5033a to 5033c in accordance with a distance from the tip end 31a of the rigid scope 3 to the target object based on a detection result of the TOF sensor 25.

With this configuration, in the medical system 5020, the joint units 5033a to 5033c can be controlled by the arm control device 5045 in accordance with the distance from the tip end 31a of the rigid scope 3 to the target object based on the detection result of the TOF sensor 25. As a result, in the medical system 5020, it is possible to improve safety since the rigid scope 3 can automatically avoid collision in a body cavity by the support arm 5027.

A distance measuring method of the endoscope system 1 is a distance calculation method of the endoscope system 1 including: the imaging device 2 including the TOF sensor 25 capable of detecting a distance to a target object; the rigid scope 3 having the illumination optical system 34 and the imaging optical system 35 and replaceably mounted to the imaging device 2; and the light source device 4 provided outside the imaging device 2 and the rigid scope 3, and connected to the illumination optical system 34 of the rigid scope 3 via the light guide 6. The distance measuring method includes: a step of acquiring each optical distance of the illumination optical system 34 and the imaging optical system 35 of the rigid scope 3, the light guide 6, the light source device 4, and the imaging device 2; a step of causing the light source device 4 to emit infrared light; and a step of calculating a distance from the tip end 31a of the rigid scope 3 to the target object on the basis of the acquired optical distance and a detection result of the TOF sensor 25.

With this configuration, in the distance measuring method, it is possible to acquire the optical distance and calculate the distance D6 from the tip end 31a of the mounted rigid scope 3 to the subject M even when the rigid scope 3 to be mounted to the imaging device 2 is changed. As a result, since a wide variety of the rigid scopes 3 can be used as the distance measuring method, the commercial value of the endoscope system 1 can be improved.

The distance measuring method further includes: a step of causing the light source device 4 to emit infrared light in a state where the jig 9 that positions the reflection unit 91 at a location at a predetermined distance from the tip end 31a of the rigid scope 3 is mounted; a step of calculating the sum of the optical distances of the rigid scope 3 and the light guide 6 on the basis of a detection result of the TOF sensor 25 in the state where the jig 9 is mounted; and a step of calculating a distance from the tip end 31a of the rigid scope 3 to the target object on the basis of a detection result of the TOF sensor 25 in the state where the jig 9 is not mounted and the sum of the optical distances.

With this configuration, in the distance measuring method, even if the rigid scope 3 or the light guide 6 is changed, the distance D6 from the tip end 31a of the rigid scope 3 to the subject M can be calculated on the basis of the detection result of the TOF sensor 25. As a result, in the distance measuring method, since a wide variety of the rigid scopes 3 and the light guides 6 can be used, it is possible to further improve the commercial value of the endoscope system 1.

The distance measuring method further includes: a step of causing the light source device 4 to emit infrared light in a state where the jig 9 that positions the reflection unit 91 at a location at a predetermined distance from the tip end 31a of the rigid scope 3 is mounted; a step of creating the correction information 504F on the basis of a predetermined distance and a distance from the tip end 31a of the rigid scope 3 to a target object, on the basis of a detection result of the TOF sensor 25 in the state where the jig 9 is mounted; and a step of calculating a distance from the tip end 31a of the rigid scope 3 to the target object, on the basis of a detection result of the TOF sensor 25 in the state where the jig 9 is not mounted and the correction information 504F.

With this configuration, in the distance measuring method, in a case where there is an error between the optical distance stored in advance in the endoscope system 1 and an actual optical distance, the optical distance can be adjusted by using the correction information 504F. As a result, in the distance measuring method, even when there is an error between the optical distance stored in advance in the endoscope system 1 and an actual optical distance, the distance D6 can be calculated with higher accuracy.

Note that the following configurations are also within the technical scope of the present disclosure.

(1)
A medical observation system including:

an imaging device including an imaging element, and a time-of-flight sensor capable of detecting a distance to a target object;
a lens barrel having an illumination optical system and an imaging optical system, the lens barrel being replaceably mounted to the imaging device; and
a light source device connected to the illumination optical system of the lens barrel via a light guide, in which the light source device includes a first light source configured to emit first light that is guided in the illumination optical system and illuminates the target object, and a second light source configured to emit second light that is guided in the illumination optical system and is to be detected by the time-of-flight sensor, and
the time-of-flight sensor receives the second light that has been reflected by the target object and passed through the imaging optical system of the lens barrel.

(2)
The medical observation system according to (1) described above, further including:

a control device configured to control the light source device, in which
the control device calculates a distance from a tip end of the lens barrel to the target object on the basis of a detection result of the time-of-flight sensor.

(3)
The medical observation system according to (2) described above, in which
the lens barrel guides the second light from the second light source by the illumination optical system, to emit the second light toward the target object.

(4)
The medical observation system according to (2) or (3), in which
the control device executes a notification process regarding a distance to the target object, on the basis of the calculated distance to the target object.

(5)
The medical observation system according to (4) described above, in which
the control device notifies of a distance to the target object.

(6)
The medical observation system according to any one of (2) to (5), in which
in a case where the calculated distance to the target object becomes closer than a threshold value, the control device executes processing for notifying that the lens barrel is approaching the target object.

(7)
The medical observation system according to any one of (2) to (6), in which
the control device causes the first light source and the second light source to emit light at different timings.

(8)
The medical observation system according to any one of (2) to (7), in which

the control device
further includes a communication unit configured to communicate with at least one of the light source device or the imaging device, and
synchronizes, via the communication unit, a timing at which the time-of-flight sensor and the second light source emit light.

(9)
The medical observation system according to any one of (2) to (8), in which
the control device further includes:

an acquisition unit configured to acquire an optical distance of each of the illumination optical system and the imaging optical system of the lens barrel, the light guide, the light source device, and the imaging device; and
a calculation unit configured to calculate a distance from the tip end of the lens barrel to the target object, on the basis of the optical distance acquired by the acquisition unit and on the basis of a detection result of the time-of-flight sensor.

(10)
The medical observation system according to any one of (2) to (9), in which

the control device further includes a storage unit configured to store optical distance information indicating the optical distance of at least one of the lens barrel or the light guide of a plurality of types, and
the acquisition unit acquires the optical distance of at least one of the lens barrel or the light guide, from the optical distance information stored in the storage unit.

(11)
The medical observation system according to any one of (2) to (9), further including:

a jig configured to position a reflection unit at a location at a predetermined distance from the tip end of the lens barrel, in which
the control device further includes a second calculation unit configured to calculate a sum of the optical distances of the lens barrel and the light guide, on the basis of a detection result of the time-of-flight sensor using the reflection unit of the jig, the predetermined distance, and the optical distances of the light source device and the imaging device, and
the acquisition unit acquires the optical distance on the basis of a sum of the optical distances calculated by the second calculation unit.

(12)
The medical observation system according to (11) described above, in which

the control device further includes a creation unit configured to create correction information on the basis of a distance from the tip end of the lens barrel to the target object, the distance being calculated by the calculation unit, and on the basis of the predetermined distance, in a case where the reflection unit is positioned at a location at the predetermined distance from the tip end of the lens barrel by the jig, and
the acquisition unit acquires the optical distance on the basis of the correction information created by the creation unit.

(13)
A medical system including:

a medical observation system including: an imaging device including an imaging element, and a time-of-flight sensor capable of detecting a distance to a target object; a lens barrel having an illumination optical system and an imaging optical system, the lens barrel being replaceably mounted to the imaging device; and a light source device connected to the illumination optical system of the lens barrel via a light guide; and
a support arm having a plurality of links rotatably connected by a joint unit, the support arm being configured to be able to hold the imaging device, in which
the light source device includes a first light source configured to emit first light that is guided in the illumination optical system and illuminates the target object, and a second light source configured to emit second light that is guided in the illumination optical system and is to be detected by the time-of-flight sensor, and
the time-of-flight sensor receives the second light that has been reflected by the target object and passed through the imaging optical system of the lens barrel.

(14)
The medical system according to (13) described above, further including:

an arm control device configured to control the support arm, in which
the arm control device controls the joint unit in accordance with a distance from the tip end of the lens barrel to the target object based on a detection result of the time-of-flight sensor.

(15)

A distance measuring method of a medical observation system including: an imaging device including an imaging element, and a time-of-flight sensor capable of detecting a distance to a target object; a lens barrel having an illumination optical system and an imaging optical system, the lens barrel being replaceably mounted to the imaging device; and a light source device connected to the illumination optical system of the lens barrel via a light guide, the distance measuring method including:

a step of acquiring an optical distance of each of the illumination optical system and the imaging optical system of the lens barrel, the light guide, the light source device, and the imaging device;
a step of causing the light source device to emit light; and
a step of calculating a distance from a tip end of the lens barrel to the target object, on the basis of the acquired optical distance and a detection result of the time-of-flight sensor.

(16)
The distance measuring method according to (15) described above, further including:

a step of causing the light source device to emit light in a state where a jig configured to position a reflection unit at a location at a predetermined distance from the tip end of the lens barrel is mounted; and
a step of calculating a sum of optical distances of the lens barrel and the light guide, on the basis of a detection result of the time-of-flight sensor in a state where the jig is mounted; and
a step of calculating a distance from the tip end of the lens barrel to the target object, on the basis of a detection result of the time-of-flight sensor in a state where the jig is not mounted and on the basis of a sum of the optical distances.

(17)
The distance measuring method according to (15) described above, further including:

a step of causing the light source device to emit light in a state where a jig configured to position a reflection unit at a location at a predetermined distance from the tip end of the lens barrel is mounted; and
a step of creating correction information on the basis of a distance from the tip end of the lens barrel to the target object and on the basis of the predetermined distance, on the basis of a detection result of the time-of-flight sensor in a state where the jig is mounted; and
a step of calculating a distance from the tip end of the lens barrel to the target object, on the basis of a detection result of the time-of-flight sensor in a state where the jig is not mounted and on the basis of the correction information.

REFERENCE SIGNS LIST

1       Endoscope system
2       Imaging device
3       Rigid scope
4       Light source device
5       Control device
6       Light guide
7       Transmission cable
8       Transmission cable
9       Jig
23      First imaging element
24      Second imaging element
25      TOF sensor
31      Insertion unit
31a     Tip end
32      Connection unit
33      Mounting unit
34      Illumination optical system
35      Imaging optical system
41      First light source

## EP 3 916 463 A1

| 42 | Second light source |
|---|---|
| 91 | Reflection unit |
| 92 | Positioning unit |
| 201 | Communication unit |
| 202 | Storage unit |
| 202A | Optical distance information |
| 203 | Control unit |
| 301 | Communication unit |
| 302 | Storage unit |
| 302A | Optical distance information |
| 303 | Control unit |
| 401 | Communication unit |
| 402 | Storage unit |
| 402A | Optical distance information |
| 403 | Control unit |
| 501 | Input unit |
| 502 | Output unit |
| 503 | Communication unit |
| 504 | Storage unit |
| 504A | Optical distance information |
| 504F | Correction information |
| 504P | Control program |
| 504T | Optical distance information |
| 505 | Control unit |
| 505A | Acquisition unit |
| 505B | Drive control unit |
| 505C | Calculation unit |
| 505D | Processing unit |
| 505E | Second calculation unit |
| 505F | Creation unit |
| 5020 | Medical system |
| 5027 | Support arm |
| 5045 | Arm control device |
| D6 | Distance |
| D7 | Reference distance |
| L1 to L5 | Optical distance |
| M | Subject |

**Claims**

1. A medical observation system comprising:

   an imaging device including an imaging element, and a time-of-flight sensor capable of detecting a distance to a target object;
   a lens barrel having an illumination optical system and an imaging optical system, the lens barrel being replaceably mounted to the imaging device; and
   a light source device connected to the illumination optical system of the lens barrel via a light guide, wherein
   the light source device includes a first light source configured to emit first light that is guided in the illumination optical system and illuminates the target object, and a second light source configured to emit second light that is guided in the illumination optical system and is to be detected by the time-of-flight sensor, and
   the time-of-flight sensor receives the second light that has been reflected by the target object and passed through the imaging optical system of the lens barrel.

2. The medical observation system according to claim 1, further comprising:

   a control device configured to control the light source device, wherein
   the control device calculates a distance from a tip end of the lens barrel to the target object on a basis of a detection result of the time-of-flight sensor.

3. The medical observation system according to claim 2, wherein
the lens barrel guides the second light from the second light source by the illumination optical system, to emit the second light toward the target object.

4. The medical observation system according to claim 3, wherein
the control device executes a notification process regarding a distance to the target object, on a basis of the calculated distance to the target object.

5. The medical observation system according to claim 4, wherein
the control device notifies of a distance to the target object.

6. The medical observation system according to claim 4, wherein
in a case where the calculated distance to the target object becomes closer than a threshold value, the control device executes processing for notifying that the lens barrel is approaching the target object.

7. The medical observation system according to claim 2, wherein
the control device causes the first light source and the second light source to emit light at different timings.

8. The medical observation system according to claim 7, wherein

the control device
further includes a communication unit configured to communicate with at least one of the light source device or the imaging device, and
synchronizes, via the communication unit, a timing at which the time-of-flight sensor and the second light source emit light.

9. The medical observation system according to claim 2, wherein
the control device further includes:

an acquisition unit configured to acquire an optical distance of each of the illumination optical system and the imaging optical system of the lens barrel, the light guide, the light source device, and the imaging device; and
a calculation unit configured to calculate a distance from the tip end of the lens barrel to the target object, on a basis of the optical distance acquired by the acquisition unit and on a basis of a detection result of the time-of-flight sensor.

10. The medical observation system according to claim 9, wherein

the control device further includes a storage unit configured to store optical distance information indicating the optical distance of at least one of the lens barrel or the light guide of a plurality of types, and
the acquisition unit acquires the optical distance of at least one of the lens barrel or the light guide, from the optical distance information stored in the storage unit.

11. The medical observation system according to claim 9, further comprising:

a jig configured to position a reflection unit at a location at a predetermined distance from the tip end of the lens barrel, wherein
the control device further includes a second calculation unit configured to calculate a sum of the optical distances of the lens barrel and the light guide, on a basis of a detection result of the time-of-flight sensor using the reflection unit of the jig, the predetermined distance, and the optical distances of the light source device and the imaging device, and
the acquisition unit acquires the optical distance on a basis of a sum of the optical distances calculated by the second calculation unit.

12. The medical observation system according to claim 11, wherein

the control device further includes a creation unit configured to create correction information on a basis of a distance from the tip end of the lens barrel to the target object, the distance being calculated by the calculation unit, and on a basis of the predetermined distance, in a case where the reflection unit is positioned at a location at the predetermined distance from the tip end of the lens barrel by the jig, and

the acquisition unit acquires the optical distance on a basis of the correction information created by the creation unit.

13. A medical system comprising:

a medical observation system including: an imaging device including an imaging element, and a time-of-flight sensor capable of detecting a distance to a target object; a lens barrel having an illumination optical system and an imaging optical system, the lens barrel being replaceably mounted to the imaging device; and a light source device connected to the illumination optical system of the lens barrel via a light guide; and

a support arm having a plurality of links rotatably connected by a joint unit, the support arm being configured to be able to hold the imaging device, wherein

the light source device includes a first light source configured to emit first light that is guided in the illumination optical system and illuminates the target object, and a second light source configured to emit second light that is guided in the illumination optical system and is to be detected by the time-of-flight sensor, and

the time-of-flight sensor receives the second light that has been reflected by the target object and passed through the imaging optical system of the lens barrel.

14. The medical system according to claim 13, further comprising:

an arm control device configured to control the support arm, wherein

the arm control device controls the joint unit in accordance with a distance from the tip end of the lens barrel to the target object based on a detection result of the time-of-flight sensor.

15. A distance measuring method of a medical observation system including: an imaging device including an imaging element, and a time-of-flight sensor capable of detecting a distance to a target object; a lens barrel having an illumination optical system and an imaging optical system, the lens barrel being replaceably mounted to the imaging device; and a light source device connected to the illumination optical system of the lens barrel via a light guide, the distance measuring method comprising:

a step of acquiring an optical distance of each of the illumination optical system and the imaging optical system of the lens barrel, the light guide, the light source device, and the imaging device;

a step of causing the light source device to emit light; and

a step of calculating a distance from a tip end of the lens barrel to the target object on a basis of the acquired optical distance and a detection result of the time-of-flight sensor.

16. The distance measuring method according to claim 15, further comprising:

a step of causing the light source device to emit light in a state where a jig configured to position a reflection unit at a location at a predetermined distance from the tip end of the lens barrel is mounted;

a step of calculating a sum of optical distances of the lens barrel and the light guide, on a basis of a detection result of the time-of-flight sensor in a state where the jig is mounted; and

a step of calculating a distance from the tip end of the lens barrel to the target object, on a basis of a detection result of the time-of-flight sensor in a state where the jig is not mounted and on a basis of a sum of the optical distances.

17. The distance measuring method according to claim 15, further comprising:

a step of causing the light source device to emit light in a state where a jig configured to position a reflection unit at a location at a predetermined distance from the tip end of the lens barrel is mounted;

a step of creating correction information on a basis of a distance from the tip end of the lens barrel to the target object and on a basis of the predetermined distance, on a basis of a detection result of the time-of-flight sensor in a state where the jig is mounted; and

a step of calculating a distance from the tip end of the lens barrel to the target object, on a basis of a detection result of the time-of-flight sensor in a state where the jig is not mounted and on a basis of the correction information.

# FIG. 1

# FIG. 2

# FIG. 3

# FIG. 4

| | | 43 | | 44 | 40 |
|---|---|---|---|---|---|

46  45

| LIGHT SOURCE BLUE | LIGHT SOURCE GREEN | LIGHT SOURCE RED | LIGHT SOURCE INFRARED (TOF) |
|---|---|---|---|

41  42

## FIG. 5

EP 3 916 463 A1

# FIG. 6

RIGID SCOPE — 3

CONTROL UNIT — 303

STORAGE UNIT — 302
OPTICAL DISTANCE INFORMATION — 302A

COMMUNICATION UNIT — 301

LIGHT SOURCE DEVICE — 4

FIRST LIGHT SOURCE — 41

CONTROL UNIT — 403

COMMUNICATION UNIT — 401

SECOND LIGHT SOURCE — 42

STORAGE UNIT — 402
OPTICAL DISTANCE INFORMATION — 402A

— 8

IMAGING DEVICE — 2

FIRST IMAGING ELEMENT — 23

SECOND IMAGING ELEMENT — 24

TOF SENSOR — 25

COMMUNICATION UNIT — 201

CONTROL UNIT — 203

STORAGE UNIT — 202
OPTICAL DISTANCE INFORMATION — 202A

— 7

CONTROL DEVICE — 5

INPUT UNIT — 501

OUTPUT UNIT — 502

COMMUNICATION UNIT — 503

STORAGE UNIT — 504
CONTROL PROGRAM — 504P
OPTICAL DISTANCE INFORMATION — 504A

CONTROL UNIT — 505
ACQUISITION UNIT — 505A
DRIVE CONTROL UNIT — 505B
CALCULATION UNIT — 505C
PROCESSING UNIT — 505D

— 1

# FIG. 7

```
                        START

                                          S101
        ACQUIRE OPTICAL DISTANCE

                                          S102
        CONTROL SYNCHRONIZATION BETWEEN
        LIGHT SOURCE DEVICE AND IMAGING DEVICE

                                          S103
        CALCULATE DISTANCE FROM TIP END OF RIGID SCOPE TO SUBJECT,
        ON BASIS OF DETECTION RESULT OF TOF SENSOR

                                          S104
        STORE CALCULATED DISTANCE IN STORAGE UNIT

                                          S105
              HAS DISTANCE                          No
        BECOME CLOSER THAN THRESHOLD
                 VALUE?

                      Yes              S106
        EXECUTE PROCESSING FOR NOTIFYING THAT
        RIGID SCOPE IS APPROACHING SUBJECT

                                          S107
                                                    No
                 END?

                      Yes

                        END
```

# FIG. 8

504T

| IDENTIFICATION INFORMATION | OPTICAL DISTANCE |
|---|---|
| ILLUMINATION OPTICAL SYSTEM OF RIGID SCOPE 3A | L3A |
| IMAGING OPTICAL SYSTEM OF RIGID SCOPE 3A | L4A |
| ILLUMINATION OPTICAL SYSTEM OF RIGID SCOPE 3B | L3B |
| IMAGING OPTICAL SYSTEM OF RIGID SCOPE 3B | L4B |
| ILLUMINATION OPTICAL SYSTEM OF RIGID SCOPE 3C | L3C |
| IMAGING OPTICAL SYSTEM OF RIGID SCOPE 3C | L4C |
| ILLUMINATION OPTICAL SYSTEM OF RIGID SCOPE 3D | L3D |
| IMAGING OPTICAL SYSTEM OF RIGID SCOPE 3D | L4D |
| IMAGING DEVICE | L5 |
| LIGHT SOURCE INFORMATION | L1 |
| LIGHT GUIDE | L2 |

FIG. 9

# FIG. 10

```
CONTROL DEVICE                                            ⌐5

  ⌐501                                                    ⌐505
┌─────────────┐                    ┌──────────────────────────────────────┐
│ INPUT UNIT  │──────────┐         │          CONTROL UNIT                │
└─────────────┘          │         │   ⌐505A              ⌐505E           │
  ⌐502                   │         │ ┌──────────────┐  ┌────────────────┐ │
┌─────────────┐          │         │ │ ACQUISITION  │  │    SECOND      │ │
│ OUTPUT UNIT │◄─────────┤         │ │    UNIT      │  │ CALCULATION UNIT│ │
└─────────────┘          │         │ └──────────────┘  └────────────────┘ │
  ⌐503                   │         │                                      │
┌─────────────────┐      │         │   ⌐505B                              │
│ COMMUNICATION   │◄─────►│         │ ┌──────────────┐                     │
│      UNIT       │       │         │ │    DRIVE     │                     │
└─────────────────┘      │         │ │ CONTROL UNIT │                     │
  ⌐504                   │         │ └──────────────┘                     │
┌─────────────────┐      │         │                                      │
│  STORAGE UNIT   │      │         │   ⌐505C                              │
│   ⌐504P         │      │         │ ┌──────────────┐                     │
│ ┌─────────────┐ │◄─────►│         │ │ CALCULATION  │                     │
│ │   CONTROL   │ │      │         │ │    UNIT      │                     │
│ │  PROGRAM    │ │      │         │ └──────────────┘                     │
│ └─────────────┘ │      │         │                                      │
│   ⌐504A         │      │         │   ⌐505D                              │
│ ┌─────────────┐ │      │         │ ┌──────────────┐                     │
│ │OPTICAL DIST.│ │      │         │ │ PROCESSING   │                     │
│ │ INFORMATION │ │      │         │ │    UNIT      │                     │
│ └─────────────┘ │      │         │ └──────────────┘                     │
└─────────────────┘      │         └──────────────────────────────────────┘
```

# FIG. 11

```
              ┌──────────┐
              │  START   │
              └────┬─────┘
                   ▼ ◄──────────────────────┐
         ╱─────────────────────────╲   ⌐S201│
        ╱  HAS JIG BEEN MOUNTED?     ╲───────┘ No
        ╲                            ╱
         ╲──────────┬──────────────╱
                    │ Yes    ⌐S202
        ┌───────────▼───────────────────────────┐
        │  CONTROL DRIVING OF LIGHT SOURCE DEVICE│
        └───────────┬───────────────────────────┘
                    │        ⌐S203
        ┌───────────▼───────────────────────────┐
        │ CALCULATE SUM OF OPTICAL DISTANCES OF  │
        │ RIGID SCOPE AND LIGHT GUIDE, ON BASIS  │
        │ OF DETECTION RESULT OF TOF SENSOR      │
        └───────────┬───────────────────────────┘
                    │        ⌐S204
        ┌───────────▼───────────────────────────┐
        │ STORE CALCULATED SUM OF OPTICAL        │
        │ DISTANCES IN STORAGE UNIT              │
        └───────────┬───────────────────────────┘
                    ▼
              ┌──────────┐
              │   END    │
              └──────────┘
```

# FIG. 12

START

S301
ACQUIRE OPTICAL DISTANCE AND SUM OF OPTICAL DISTANCES

S102
CONTROL SYNCHRONIZATION BETWEEN
LIGHT SOURCE DEVICE AND IMAGING DEVICE

S311
CALCULATE DISTANCE FROM TIP END OF RIGID SCOPE TO SUBJECT,
ON BASIS OF DETECTION RESULT OF TOF SENSOR,
OPTICAL DISTANCE, AND SUM OF OPTICAL DISTANCES

S104
STORE CALCULATED DISTANCE IN STORAGE UNIT

S105
HAS DISTANCE
BECOME CLOSER THAN THRESHOLD
VALUE?
No

Yes

S106
EXECUTE PROCESSING FOR NOTIFYING THAT
RIGID SCOPE IS APPROACHING SUBJECT

S107
END?
No

Yes

END

# FIG. 13

5

**CONTROL DEVICE**

501

INPUT UNIT

502

OUTPUT UNIT

503

COMMUNICATION UNIT

504

STORAGE UNIT

504P

CONTROL PROGRAM

504F

CORRECTION
INFORMATION

505

**CONTROL UNIT**

505A

ACQUISITION UNIT

505F

CREATION UNIT

505B

DRIVE
CONTROL UNIT

505C

CALCULATION UNIT

505D

PROCESSING UNIT

# FIG. 14

```
                    START

                      │
                      ▼
              ┌───────────────┐        S201
         ◄────┤ HAS JIG BEEN  ├──────  No
              │   MOUNTED?    │
              └───────────────┘
                      │ Yes
                      ▼                  S202
    ┌─────────────────────────────────────────┐
    │  CONTROL DRIVING OF LIGHT SOURCE DEVICE  │
    └─────────────────────────────────────────┘
                      │                  S411
                      ▼
    ┌─────────────────────────────────────────┐
    │  CALCULATE ACTUAL OPTICAL DISTANCE ON BASIS OF │
    │ DETECTION RESULT OF TOF SENSOR AND REFERENCE DISTANCE │
    └─────────────────────────────────────────┘
                      │                  S412
                      ▼
    ┌─────────────────────────────────────────┐
    │ CREATE CORRECTION INFORMATION ON BASIS OF CALCULATION RESULT │
    └─────────────────────────────────────────┘
                      │                  S413
                      ▼
    ┌─────────────────────────────────────────┐
    │ STORE CREATED CORRECTION INFORMATION IN STORAGE UNIT │
    └─────────────────────────────────────────┘
                      │
                      ▼
                     END
```

# FIG. 15

START

ACQUIRE CORRECTION INFORMATION FROM STORAGE UNIT ⸰S501

CONTROL SYNCHRONIZATION BETWEEN
LIGHT SOURCE DEVICE AND IMAGING DEVICE ⸰S102

CALCULATE DISTANCE FROM TIP END OF RIGID SCOPE TO SUBJECT,
ON BASIS OF DETECTION RESULT OF TOF SENSOR
AND CORRECTION INFORMATION ⸰S511

STORE CALCULATED DISTANCE IN STORAGE UNIT ⸰S104

HAS DISTANCE
BECOME CLOSER THAN THRESHOLD
VALUE? ⸰S105    No

Yes

EXECUTE PROCESSING FOR NOTIFYING THAT
RIGID SCOPE IS APPROACHING SUBJECT ⸰S106

END? ⸰S107    No

Yes

END

## INTERNATIONAL SEARCH REPORT

| International application No. |
|---|
| PCT/JP2020/000149 |

### A. CLASSIFICATION OF SUBJECT MATTER

Int. Cl. G02B23/26(2006.01)i, G01C3/06(2006.01)i, A61B1/00(2006.01)i, A61B1/06(2006.01)i
FI: A61B1/00 553, A61B1/06 611, A61B1/00 650, A61B1/00 630, A61B1/00 655, G01C3/06 120Q, G02B23/26 D

According to International Patent Classification (IPC) or to both national classification and IPC

### B. FIELDS SEARCHED

Minimum documentation searched (classification system followed by classification symbols)
Int. Cl. A61B1/00-1/32, G02B23/24-23/26, G01C3/00-3/32

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y<br>A | WO 2018/159328 A1 (SONY CORP.) 07 September 2018, paragraphs [0013]-[0097], [0100]-[0109], [0132]-[0153], fig. 1-4, 11-20, paragraphs [0013]-[0097], [0100]-[0109], [0132]-[0153], fig. 1-4, 11-20 | 1-10, 13-15<br>11, 12, 16, 17 |
| Y | WO 2017/217498 A1 (TOKYO UNIVERSITY OF AGRICULTURE AND TECHNOLOGY) 21 December 2017, paragraphs [0048], [0071]-[0080], fig. 1, 5 | 1-10, 13-15 |
| A | JP 2017-176811 A (SONY CORP.) 05 October 2017, entire text, all drawings | 1-17 |
| A | JP 2017-205492 A (PANASONIC INTELLECTUAL PROPERTY MANAGEMENT CO., LTD.) 24 November 2017, entire text, all drawings | 1-17 |

☐ Further documents are listed in the continuation of Box C.　　☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>03.03.2020 | Date of mailing of the international search report<br>17.03.2020 |
|---|---|
| Name and mailing address of the ISA/<br>　　Japan Patent Office<br>　　3-4-3, Kasumigaseki, Chiyoda-ku,<br>　　Tokyo 100-8915, Japan | Authorized officer<br><br><br>Telephone No. |

Form PCT/ISA/210 (second sheet) (January 2015)

**INTERNATIONAL SEARCH REPORT**
Information on patent family members

| International application No. |
|---|
| PCT/JP2020/000149 |

| Patent Documents referred to in the Report | Publication Date | Patent Family | Publication Date |
|---|---|---|---|
| WO 2018/159328 A1 | 07.09.2018 | CN 110325093 A paragraphs [0041]-[0150], [0153]-[0162], [0195]-[0224], fig. 1-4, 11-20 | |
| WO 2017/217498 A1 | 21.12.2017 | (Family: none) | |
| JP 2017-176811 A | 05.10.2017 | US 2018/0360299 A1 entire document WO 2017/169335 A1 | |
| JP 2017-205492 A | 24.11.2017 | (Family: none) | |

Form PCT/ISA/210 (patent family annex) (January 2015)

**EP 3 916 463 A1**

REFERENCES CITED IN THE DESCRIPTION

**Patent documents cited in the description**

- JP 2017176811 A